# NEW EUROPEAN PATENT SPECIFICATION

(11) **EP 2 749 254 B2**
(45) Date of publication and mention of the opposition decision: **06.11.2019**
(45) Mention of the grant of the patent: 17.06.2015
(21) Application number: 14161991.6
(22) Date of filing: 22.12.2004
(51) Int. Cl.: A61F 2/01, A61F 2/24

(54) **Repositionable heart valve**
Umpositionierbare Herzklappe
Valvule cardiaque repositionnable

(30) Priority: 23.12.2003 US 746280; 23.12.2003 US 746942; 23.12.2003 US 746240; 23.12.2003 US 746872; 23.12.2003 US 746887; 23.12.2003 US 746120; 23.12.2003 US 746285; 15.07.2004 US 893151; 15.07.2004 US 893131; 15.07.2004 US 893143; 15.07.2004 US 893142; 21.10.2004 US 972287; 21.10.2004 US 971535; 05.11.2004 US 982692; 05.11.2004 US 982388
(43) Date of publication of application: 02.07.2014
(62) Divisional of application: 04815634.3
(73) Proprietor: Boston Scientific Scimed, Inc., Maple Grove, MN 55311-1566 (US)
(72) Inventor: Salahieh, Amr, Saratoga, CA California 95070 (US); Brandt, Brian, D., San Jose, CA California 95119 (US); Morejohn, Dwight, P., Davis, CA California 95616 (US); Haug, Ulrich, R., Campbell, CA California 95008 (US); Dueri, Jean-Pierre, Stockton, CA California 95219 (US); Valencia, Hans, F., San Jose, CA California 95125 (US); Geshlider, Robert, A., San Francisco, CA California 94131 (US); Krolik, Jeff, Campbell, CA California 95008 (US); Saul, Tom, Moss Beach, CA California 94038 (US); Argento, Claudio, Los Gatos, CA California 95033 (US); Hildebrand, Daniel, Menlo Park, CA California 94025 (US)
(74) Representative: Peterreins Schley

(56) References cited:
- WO-A1-00/44313
- WO-A1-00/47139
- WO-A1-01/54625
- WO-A1-95/28899
- WO-A1-98/29057
- WO-A1-98/55047
- WO-A1-03/047468
- WO-A1-03/049642
- WO-A1-03/088873
- WO-A2-01/76510
- WO-A2-03/037222
- WO-A2-2005/102015
- WO-A2-2006/005015
- FR-A1- 2 826 863
- US-A- 3 413 387
- US-A- 4 994 072
- US-A- 5 258 023
- US-A- 5 628 786
- US-A- 5 855 601
- US-A- 6 015 431
- US-A1- 2001 027 338
- US-A1- 2001 039 450
- US-A1- 2003 070 682
- US-A1- 2003 236 567
- US-A1- 2004 082 989
- US-B1- 6 245 102
- US-B1- 6 387 122
- US-B1- 6 454 799
- US-B1- 6 729 365
- US-B2- 6 352 554
- MOAZAMI, NADER ET AL: "Transluminal Aortic Valve Placement", ASAIO JOURNAL, vol. 42, 1996, pages M381-M385,
- STAHLI ET AL: "Aortic regurgitation after transcatheter aortic valve implantation: mechanisms and implications", Cardiovasc Diagn Ther, vol. 3, no. 1, 2013, pages 15-22,
- "History of Percutaneous Aortic Valve Prosthesis", Herz Kardiovaskuläre Erkrankungen,, vol. 34, no. 5, 27 August 2009 (2009-08-27), pages 343-346, XP019741761,
- GOOLEY, R. ET AL: "The Lotus transcatheter aortic valve: a next-generation repositionable, resheathable and recapturable prothesis", Interv. Cardiol., vol. 6, no. 4, 2014, pages 357-365,
- DUERIG ET AL: "A comparison of balloon- and self-expanding stents", Min Invas Ther & Allied Technol, vol. 11, no. 4, 2002, pages 173-178,
- CRIBIER, A ET AL: "Circulation December 10", , 2002, pages 3006-3008,
- DALBY, M. ET AL: BrJ Cardiol, vol. 10, 2003, pages 450-452,

## Description

### BACKGROUND OF THE INVENTION

The present invention relates to an apparatus for endovascularly replacing a heart valve comprising a seal as set forth in the claims

Heart valve surgery is used to repair or replace diseased heart valves. Valve surgery is an open-heart procedure conducted under general anesthesia. An incision is made through the patient's sternum (sternotomy), and the patient's heart is stopped while blood flow is rerouted through a heart-lung bypass machine.

Valve replacement may be indicated when there is a narrowing of the native heart valve, commonly referred to as stenosis, or when the native valve leaks or regurgitates.
When replacing the valve, the native valve is excised and replaced with either a biologic or a mechanical valve. Mechanical valves require lifelong anticoagulant medication to prevent blood clot formation, and clicking of the valve often may be heard through the chest. Biologic tissue valves typically do not require such medication. Tissue valves may be obtained from cadavers or may be porcine or bovine, and are commonly attached to synthetic rings that are secured to the patient's heart.

Valve replacement surgery is a highly invasive operation with significant concomitant risk. Risks include bleeding, infection, stroke, heart attack, arrhythmia, renal failure, adverse reactions to the anesthesia medications, as well as sudden death. 2-5% of patients die during surgery.

Post-surgery, patients temporarily may be confused due to emboli and other factors associated with the heart-lung machine. The first 2-3 days following surgery are spent in an intensive care unit where heart functions can be closely monitored. The average hospital stay is between 1 to 2 weeks, with several more weeks to months required for complete recovery.

In recent years, advancements in minimally invasive surgery and interventional cardiology have encouraged some investigators to pursue percutaneous replacement of the aortic heart valve. Percutaneous Valve Technologies ("PVT") of Fort Lee, New Jersey, has developed a balloon-expandable stent integrated with a bioprosthetic valve. The stent/valve device is deployed across the native diseased valve to permanently hold the valve open, thereby alleviating a need to excise the native valve and to position the bioprosthetic valve in place of the native valve. PVT's device is designed for delivery in a cardiac catheterization laboratory under local anesthesia using fluoroscopic guidance, thereby avoiding general anesthesia and open-heart surgery. The device was first implanted in a patient in April of 2002.

PVT's device suffers from several drawbacks. Deployment of PVT's stent is not reversible, and the stent is not retrievable. This is a critical drawback because improper positioning too far up towards the aorta risks blocking the coronary ostia of the patient. Furthermore, a misplaced stent/valve in the other direction (away from the aorta, closer to the ventricle) will impinge on the mitral apparatus and eventually wear through the leaflet as the leaflet continuously rubs against the edge of the stent/valve.

Another drawback of the PVT device is its relatively large cross-sectional delivery profile. The PVT system's stent/valve combination is mounted onto a delivery balloon, making retrograde delivery through the aorta challenging. An antegrade transseptal approach may therefore be needed, requiring puncture of the septum and routing through the mitral valve, which significantly increases complexity and risk of the procedure. Very few cardiologists are currently trained in performing a transseptal puncture, which is a challenging procedure by itself.

Other prior art replacement heart valves use self-expanding stents as anchors. In the endovascular aortic valve replacement procedure, accurate placement of aortic valves relative to coronary ostia and the mitral valve is critical. Standard self-expanding systems have very poor accuracy in deployment, however. Often the proximal end of the stent is not released from the delivery system until accurate placement is verified by fluoroscopy, and the stent typically jumps once released. It is therefore often impossible to know where the ends of the stent will be with respect to the native valve, the coronary ostia and the mitral valve.

Also, visualization of the way the new valve is functioning prior to final deployment is very desirable. Visualization prior to final and irreversible deployment cannot be done with standard self-expanding systems, however, and the replacement valve is often not fully functional before final deployment.

Another drawback of prior art self-expanding replacement heart valve systems is their lack of radial strength. In order for self-expanding systems to be easily delivered through a delivery sheath, the metal needs to flex and bend inside the delivery catheter without being plastically deformed. In arterial stents, this is not a challenge, and there are many commercial arterial stent systems that apply adequate radial force against the vessel wall and yet can collapse to a small enough of a diameter to fit inside a delivery catheter without plastically deforming.

However when the stent has a valve fastened inside it, as is the case in aortic valve replacement, the anchoring of the stent to vessel walls is significantly challenged during diastole. The force to hold back arterial pressure and prevent blood from going back inside the ventricle during diastole will be directly transferred to the stent/vessel wall interface. Therefore the amount of radial force required to keep the self expanding stent/valve in contact with the vessel wall and not sliding will be much higher than in stents that do not have valves inside of them. Moreover, a self-expanding stent without sufficient radial force will end up dilating and contracting with each heartbeat, thereby distorting the valve, affecting its function and possibly migrating and dislodging completely. Simply increasing strut thickness of the self-expanding stent is not a practical solution as it runs the risk of larger profile and/or plastic deformation of the self-expanding stent.

U.S. patent application Serial No. 2002/0151970 to Garrison et al. describes a two-piece device for replacement of the aortic valve that is adapted for delivery through a patient's aorta. A stent is percutaneously placed across the native valve, then a replacement valve is positioned within the lumen of the stent. By separating the stent and the valve during delivery, a profile of the device's delivery system may be sufficiently reduced to allow aortic delivery without requiring a transseptal approach. Both the stent and a frame of the replacement valve may be balloon-expandable or self-expanding.

While providing for an aortic approach, devices described in the Garrison patent application suffer from several drawbacks. First, the stent portion of the device is delivered across the native valve as a single piece in a single step, which precludes dynamic repositioning of the stent during delivery. Stent foreshortening or migration during expansion may lead to improper alignment.

Additionally, Garrison's stent simply crushes the native valve leaflets against the heart wall and does not engage the leaflets in a manner that would provide positive registration of the device relative to the native position of the valve. This increases an immediate risk of blocking the coronary ostia, as well as a longer-term risk of migration of the device post-implantation. Furtherstill, the stent comprises openings or gaps in which the replacement valve is seated post-delivery. Tissue may protrude through these gaps, thereby increasing a risk of improper seating of the valve within the stent.

In view of drawbacks associated with previously known techniques for percutaneously replacing a heart valve, it would be desirable to provide methods and apparatus that overcome those drawbacks.
WO 00/47139 discloses a valve implantation sysetm having a valve displacer and a replacement valve attached to the valve displacer before or after introduction

### SUMMARY OF THE INVENTION

The present invention relates to an apparatus for endovascularly replacing a heart valve comprising a seal as set forth in the claims.

### BRIEF DESCRIPTION OF THE DRAWINGS

Figures 1A-B are elevational views of a replacement heart valve and anchor according to one embodiment of the invention.
Figures 2A-B are sectional views of the anchor and valve of Figures 1.
Figures 3A-B show delivery and deployment of a replacement heart valve and anchor, such as the anchor and valve of Figures 1 and 2.
Figures 4A-F also show delivery and deployment of a replacement heart valve and anchor, such as the anchor and valve of Figures 1 and 2.
Figures 5A-I show the use of a replacement heart valve and anchor to replace an aortic valve.
Figures 6A-F show the use of a replacement heart valve and anchor with a positive registration feature to replace an aortic valve.
Figure 7 shows the the use of a replacement heart valve and anchor with an alternative positive registration feature to replace an aortic valve.
Figures 8A-C show another embodiment of a replacement heart valve and anchor according to the invention.
Figures 9A-H show delivery and deployment of the replacement heart valve and anchor of Figures 8.
Figure 10 is a cross-sectional drawing of the delivery system used with the method and apparatus of Figures 8 and 9.
Figure 11 demonstrates paravalvular leaking around a replacement heart valve and anchor.
Figure 12 shows a seal for use with a replacement heart valve and anchor of this invention.
Figures 13A-E show alternative arrangements of seals on a replacement heart valve and anchor.
Figures 14A-C show alternative seal designs for use with replacement heart valves and anchors.
Figure 15 shows yet another embodiment of the delivery and deployment apparatus of the invention in use with a replacement heart valve and anchor.
Figure 16 shows the delivery and deployment apparatus of Figure 15 in the process of deploying a replacement heart valve and anchor.
Figure 17 shows an embodiment of the invention employing seals at the interface of the replacement heart valve and anchor and the patient's tissue.
Figure 18 is a longitudinal cross-sectional view of the seal shown in Figure 17 in compressed form.
Figure 19 is a transverse cross-sectional view of the seal shown in Figure 18.
Figure 20 is a longitudinal cross-sectional view of the seal shown in Figure 17 in expanded form.
Figure 21 is a transverse cross-sectional view of the seal shown in Figure 20.
Figure 22 shows yet another embodiment of the replacement heart valve and anchor of this invention in an undeployed configuration.
Figure 23 shows the replacement heart valve and anchor of Figure 22 in a deployed configuration.
Figure 24 shows the replacement heart valve and anchor of Figures 22 and 23 deployed in a patient's heart valve.
Figures 25A and 25B show replacement valve apparatus in accordance with the present invention.

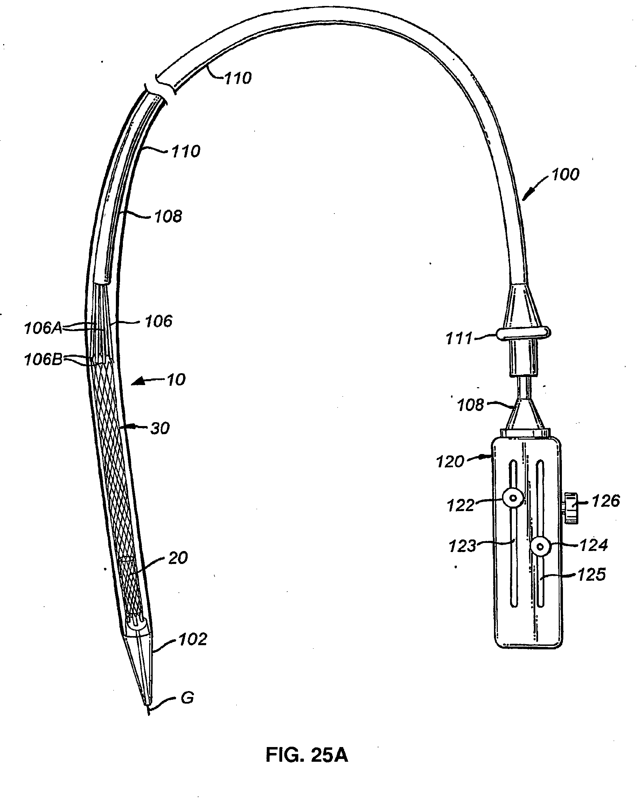

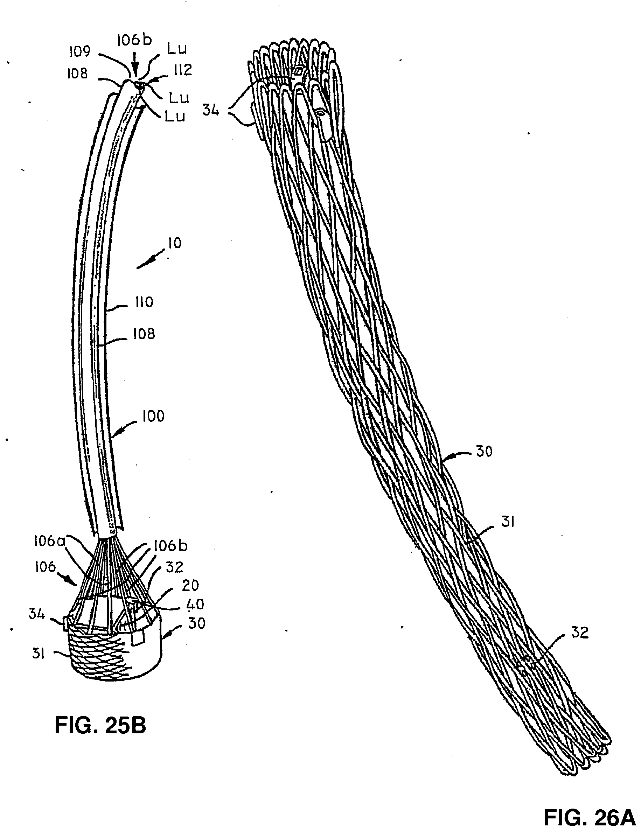

Figure 25 illustrates the apparatus in a collapsed delivery configuration within a delivery system. Figure 25B illustrates the apparatus in an expanded configuration partially deployed from the delivery system.
Figures 26A-26F show an anchor of the apparatus of Figures 25 in the collapsed delivery configuration and the expanded deployed configuration, as well as the full apparatus in the deployed configuration, and optional locking mechanisms for use with the apparatus.

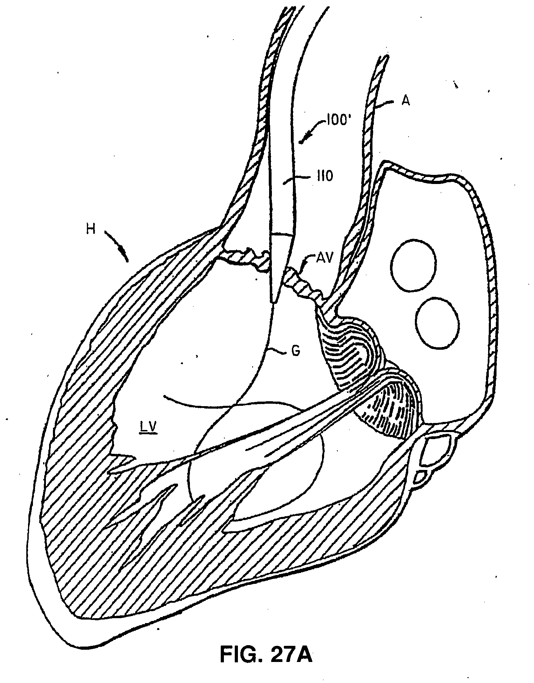

Figures 27A-27F illustrate deployment of an anchor with leaflet engagement elements on the deployment system.

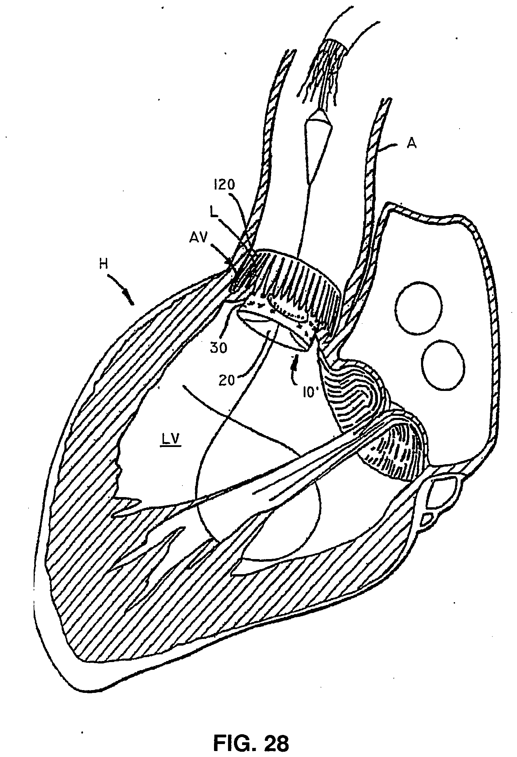

Figure 28 illustrates a deployed anchor with leaflet engagement elements on the proximal end of the anchor.

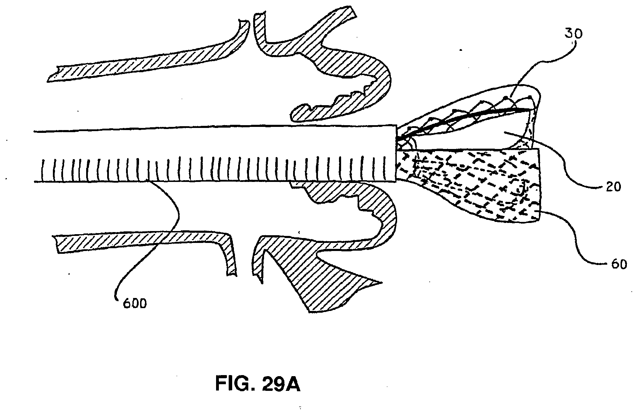

Figures 29A-29C illustrate deployment of an anchor with anchor registration elements and a seal.

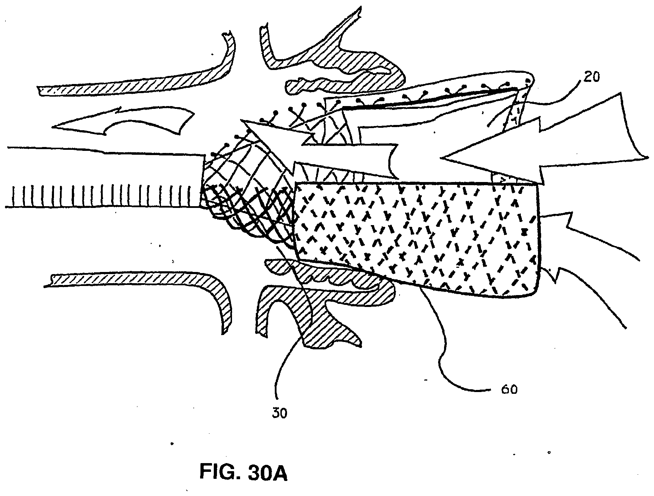

Figures 30A-30B illustrate an embodiment of the apparatus with a seal that does not reach the proximal end of the anchor during both systole and diastole.

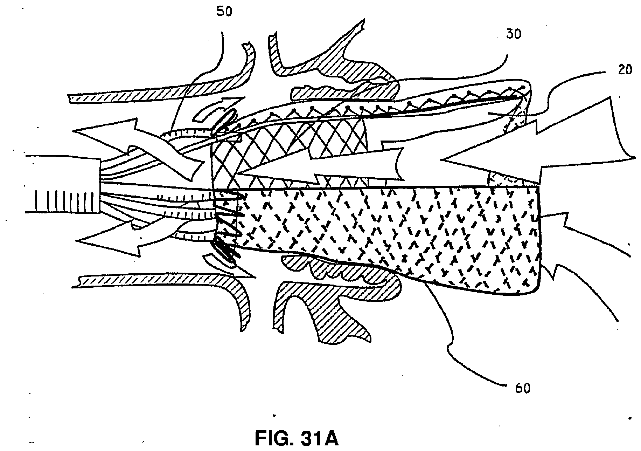

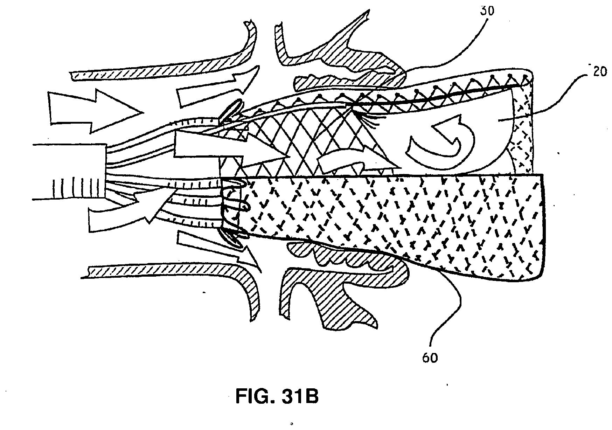

Figures 31A-31B illustrate an embodiment of the apparatus with a seal that reaches the proximal end of the anchor during both systole and diastole.

### DETAILED DESCRIPTION

The present invention relates to apparatus and methods for endovascularly or percutaneously delivering and deploying a prosthesis, e.g., an aortic prosthesis, within and/or across a patient's native heart valve, referred to hereinafter as replacing the patient's heart valve. A delivery system and/or deployment tool is provided including a sheath assembly and a guidewire for placing the prosthetic apparatus endovascularly within the patient and a user control allowing manipulation of the prosthetic apparatus from external to the patient through the application of a non-hydraulically expanding or non-pneumatically expanding force on the anchor. A hydraulically or pneumatically expanding force would be, for example, a force applied to the anchor by a balloon expanded within the anchor. In certain embodiments, the application of a non-hydraulically expanding or non-pneumatically expanding force could include the use of a hydraulic component transmitting a proximally or distally directed force on an anchor.

The apparatus includes an anchor and a replacement valve. The anchor includes an expandable anchor such as a braid. In preferred embodiments, the expandable braid includes closed edges, but the edges may alternatively be open. The replacement valve is adapted to be secured within the anchor, and as such, be delivered endovascularly to the patient's heart to replace one of the patient's native heart valves. More preferably, the apparatus and methods of the present invention contemplate replacement of the patient's aortic valve.

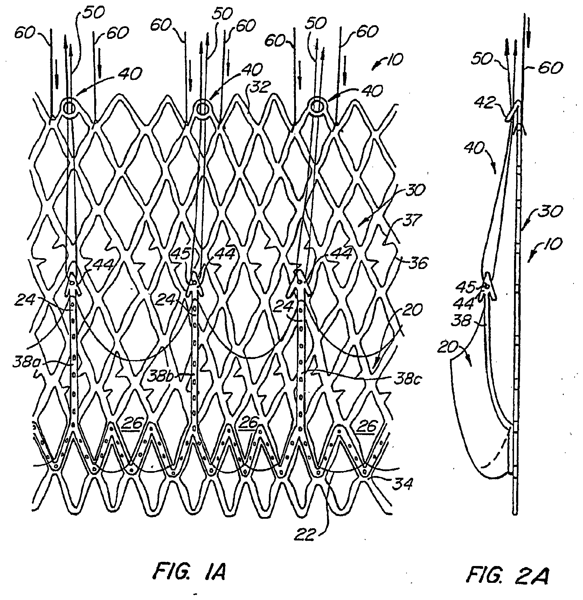

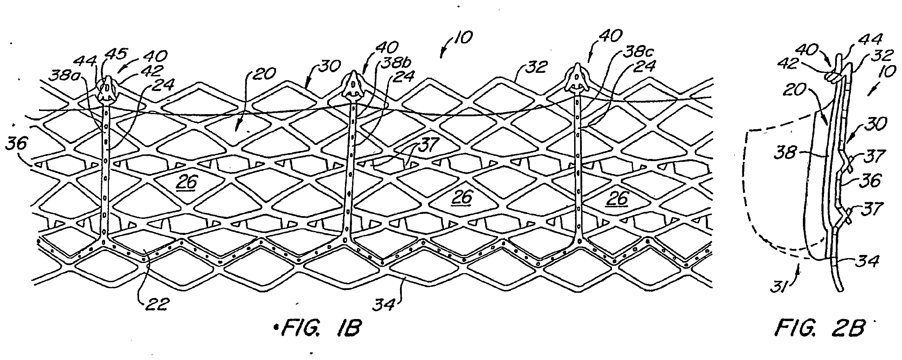

With reference now to Figures 1-4, a first embodiment of replacement heart valve apparatus in accordance with the present invention is described, including a method of actively foreshortening and expanding the apparatus from a delivery configuration and to a deployed configuration. Apparatus 10 comprises replacement valve 20 disposed within and coupled to anchor 30. Figures 1 schematically illustrate individual cells of anchor 30 of apparatus 10, and should be viewed as if the cylindrical anchor has been cut open and laid flat. Figures 2 schematically illustrate a detail portion of apparatus 10 in side-section.

Anchor 30 has a lip region 32, a skirt region 34 and a body region 36. First, second and third posts 38a, 38b and 38c, respectively, are coupled to skirt region 34 and extend within lumen 31 of anchor 30. Posts 38 preferably are spaced 120° apart from one another about the circumference of anchor 30.

Anchor 30 preferably is fabricated by using self-expanding patterns (laser cut or chemically milled), braids, and materials, such as a stainless steel, nickel-titanium ("Nitinol") or cobalt chromium but alternatively may be fabricated using balloon-expandable patterns where the anchor is designed to plastically deform to it's final shape by means of balloon expansion. Replacement valve 20 is preferably from biologic tissues, e.g. porcine valve leaflets or bovine or equine pericardium tissues, alternatively it can be made from tissue engineered materials (such as extracellular matrix material from Small Intestinal Submucosa (SIS)) but alternatively may be prosthetic from an elastomeric polymer or silicone, Nitinol or stainless steel mesh or pattern (sputtered, chemically milled or laser cut). The leaflet may also be made of a composite of the elastomeric or silicone materials and metal alloys or other fibers such Kevlar or carbon. Annular base 22 of replacement valve 20 preferably is coupled to skirt region 34 of anchor 30, while commissures 24 of replacement valve leaflets 26 are coupled to posts 38.

Anchor 30 may be actuated using external non-hydraulic or non-pneumatic force to actively foreshorten in order to increase its radial strength. As shown below, the proximal and distal end regions of anchor 30 may be actuated independently. The anchor and valve may be placed and expanded in order to visualize their location with respect to the native valve and other anatomical features and to visualize operation of the valve. The anchor and valve may thereafter be repositioned and even retrieved into the delivery sheath or catheter. The apparatus may be delivered to the vicinity of the patient's aortic valve in a retrograde approach in a catheter having a diameter no more than 23 french, preferably no more than 21 french, more preferably no more than 19 french, or more preferably no more than 17 french. Upon deployment the anchor and replacement valve capture the native valve leaflets and positively lock to maintain configuration and position.

A deployment tool is used to actuate, reposition, lock and/or retrieve anchor 30. In order to avoid delivery of anchor 30 on a balloon for balloon expansion, a non-hydraulic or non-pneumatic anchor actuator is used. In this embodiment, the actuator is a deployment tool that includes distal region control actuators 50, control actuators 60 (embodied here as rods or tubes) and proximal region control actuators 62. Locks 40 include posts or arms 38 preferably with male interlocking elements 44 extending from skirt region 34 and mating female interlocking elements 42 in lip region 32. Male interlocking elements 44 have eyelets 45. Control actuators 50 pass from a delivery system for apparatus 10 through female interlocking elements 42, through eyelets 45 of male interlocking elements 44, and back through female interlocking elements 42, such that a double strand of wire 50 passes through each female interlocking element 42 for manipulation by a medical practitioner external to the patient to actuate and control the anchor by changing the anchor's shape. Control actuators 50 may comprise, for example, strands of suture or wire.

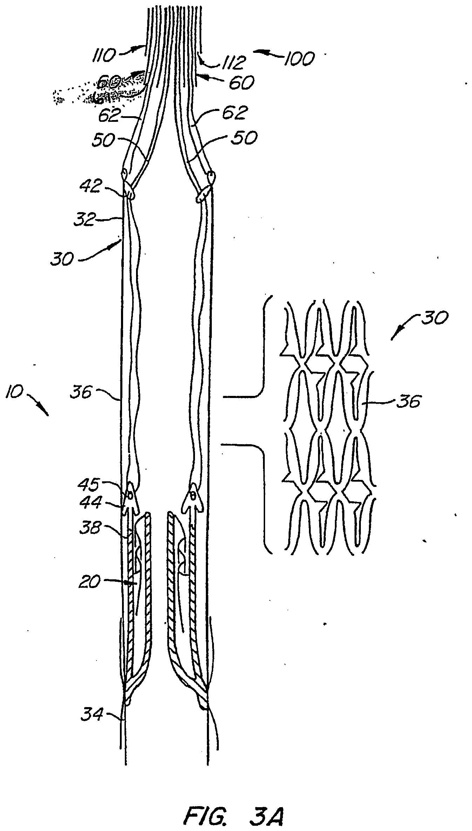

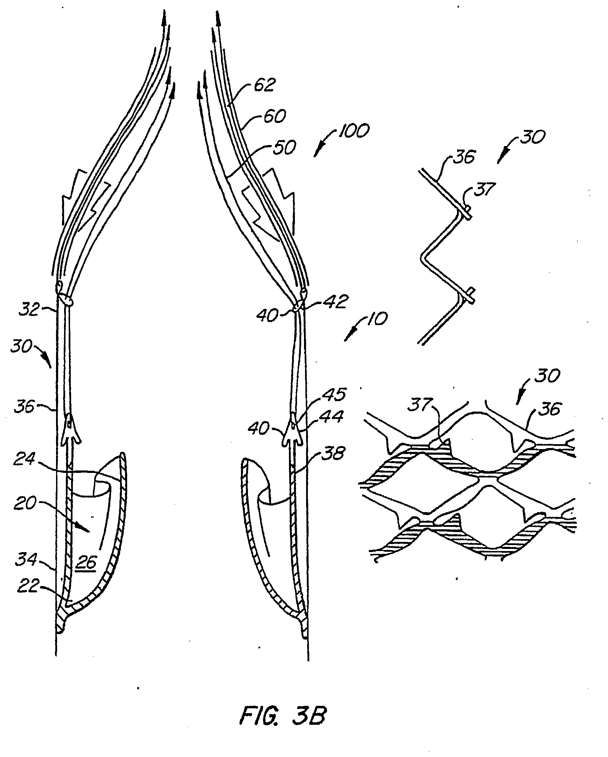

Actuators 60 are reversibly coupled to apparatus 10 and may be used in conjunction with actuators 50 to actuate anchor 30, e.g., to foreshorten and lock apparatus 10 in the fully deployed configuration. Actuators 60 also facilitate repositioning and retrieval of apparatus 10, as described hereinafter. For example, anchor 30 may be foreshortened and radially expanded by applying a distally directed force on actuators 60 while proximally retracting actuators 50. As seen in Figures 3, control actuators 62 pass through interior lumens 61 of actuators 60. This ensures that actuators 60 are aligned properly with apparatus 10 during deployment and foreshortening. Control actuators 62 can also actuate anchor 60; proximally directed forces on control actuators 62 contacts the proximal lip region 32 of anchor 30. Actuators 62 also act to couple and decouple actuators 60 from apparatus 10. Actuators 62 may comprise, for example, strands of suture or wire.

Figures 1A and 2A illustrate anchor 30 in a delivery configuration or in a partially deployed configuration (e.g., after dynamic self-expansion expansion from a constrained delivery configuration within a delivery sheath). Anchor 30 has a relatively long length and a relatively small width in the delivery or partially deployed configuration, as compared to the foreshortened and fully deployed configuration of Figures 1B and 2B.

In Figures 1A and 2A, replacement valve 20 is collapsed within lumen 31 of anchor 30. Retraction of actuators 50 relative to actuators 60 foreshortens anchor 30, which increases the anchor's width while decreasing its length. Such foreshortening also properly seats replacement valve 20 within lumen 31 of anchor 30. Imposed foreshortening will enhance radial force applied by apparatus 10 to surrounding tissue over at least a portion of anchor 30. In some embodiments, the anchor exerts an outward force on surrounding tissue to engage the tissue in such way to prevent migration of anchor caused by force of blood against closed leaflet during diastole. This anchoring force is preferably 0,454 kg to 0,907 kg [1 to 2 lbs], more preferably 0,907 kg to 1,814 kg [2 to 4 lbs], or more preferably 1,814 kg to 4,536 kg [4 to 10 lbs]. In some embodiments, the anchoring force is preferably greater than 0,454 kg [1 pound], more preferably greater than 0,907 kg [2 pounds], or more preferably greater than 1,814 kg [4 pounds]. Enhanced radial force of the anchor is also important for enhanced crush resistance of the anchor against the surrounding tissue due to the healing response (fibrosis and contraction of annulus over a longer period of time) or to dynamic changes of pressure and flow at each heart beat. In an alternative embodiment, the anchor pattern or braid is designed to have gaps or areas where the native tissue is allowed to protrude through the anchor slightly (not shown) and as the foreshortening is applied, the tissue is trapped in the anchor. This feature would provide additional means to prevent anchor migration and enhance long term stability of the device.

Deployment of apparatus 10 is fully reversible until lock 40 has been locked via mating of male interlocking elements 44 with female interlocking elements 42. Deployment is then completed by decoupling actuators 60 from lip section 32 of anchor 30 by retracting one end of each actuator 62 relative to the other end of the actuator, and by retracting one end of each actuator 50 relative to the other end of the actuator until each actuator has been removed from eyelet 45 of its corresponding male interlocking element 44.

As best seen in Figure 2B, body region 36 of anchor 30 optionally may comprise barb elements 37 that protrude from anchor 30 in the fully deployed configuration, for example, for engagement of a patient's native valve leaflets and to preclude migration of the apparatus.

With reference now to Figures 3, a delivery and deployment system for a self-expanding embodiment of apparatus 10 including a sheath 110 having a lumen 112. Self-expanding anchor 30 is collapsible to a delivery configuration within lumen 112 of sheath 110, such that apparatus 10 may be delivered via delivery system 100. As seen in Figure 3A, apparatus 10 may be deployed from lumen 112 by retracting sheath 110 relative to apparatus 10, control actuators 50 and actuators 60, which causes anchor 30 to dynamically self-expand to a partially deployed configuration. Control actuators 50 then are retracted relative to apparatus 10 and actuators 60 to impose foreshortening upon anchor 30, as seen in Figure 3B.

During foreshortening, actuators 60 push against lip region 32 of anchor 30, while actuators 50 pull on posts 38 of the anchor. Actuators 62 may be retracted along with actuators 50 to enhance the distally-directed pushing force applied by actuators 60 to lip region 32. Continued retraction of actuators 50 relative to actuators 60 would lock locks 40 and fully deploy apparatus 10 with replacement valve 20 properly seated within anchor 30, as in Figures 1B and 2B. Apparatus 10 comprises enhanced radial strength in the fully deployed configuration as compared to the partially deployed configuration of Figure 3A. Once apparatus 10 has been fully deployed, actuators 50 and 62 may be removed from apparatus 10, thereby separating delivery system 100 including actuators 60 from the apparatus.

Deployment of apparatus 10 is fully reversible until locks 40 have been actuated. For example, just prior to locking the position of the anchor and valve and the operation of the valve may be observed under fluoroscopy. If the position needs to be changed, by alternately relaxing and reapplying the proximally directed forces exerted by control actuators 50 and/or control actuators 62 and the distally directed forces exerted by actuators 60, expansion and contraction of the lip and skirt regions of anchor 30 may be independently controlled so that the anchor and valve can be moved to, e.g., avoid blocking the coronary ostia or impinging on the mitral valve. Apparatus 10 may also be completely retrieved within lumen 112 of sheath 110 by simultaneously proximally retracting actuators 50 and actuators 60/actuators 62 relative to sheath 110. Apparatus 10 then may be removed from the patient or repositioned for subsequent redeployment.

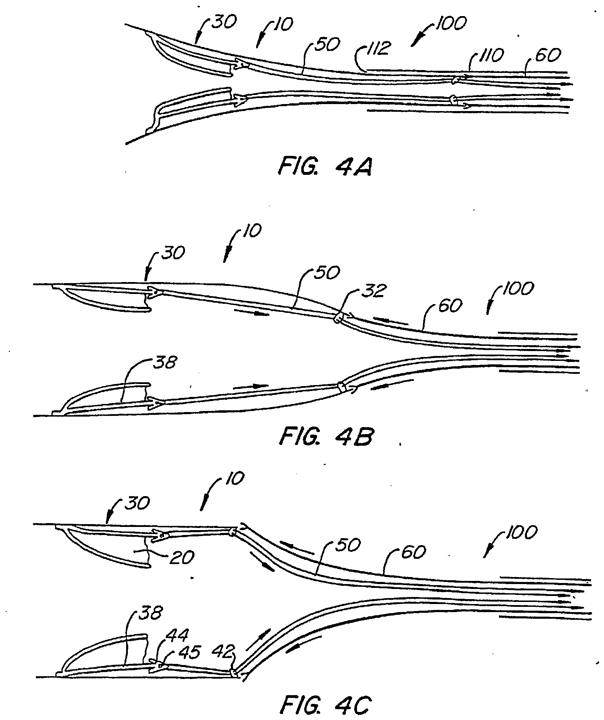

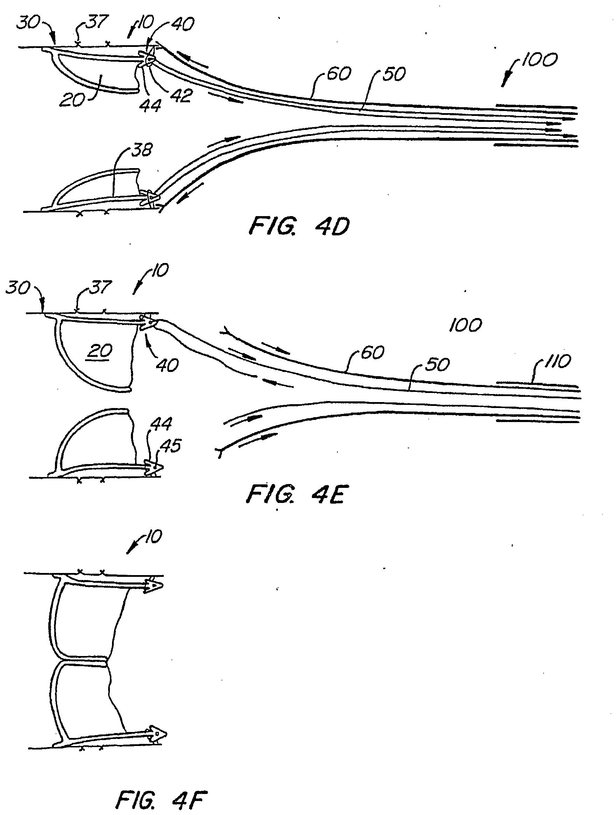

Referring now to Figures 4, step-by-step deployment of apparatus 10 via delivery system 100 is described. In Figure 4A, sheath 110 is retracted relative to apparatus 10, actuators 50 and actuators 60, thereby causing self-expandable anchor 30 to dynamically self-expand apparatus 10 from the collapsed delivery configuration within lumen 112 of sheath 110 to the partially deployed configuration. Apparatus 10 may then be dynamically repositioned via actuators 60 to properly orient the apparatus, e.g. relative to a patient's native valve leaflets.

In Figure 4B, control actuators 50 are retracted while actuators 60 are advanced, thereby urging lip region 32 of anchor 30 in a distal direction while urging posts 38 of the anchor in a proximal direction. This foreshortens apparatus 10, as seen in Figure 4C. Deployment of apparatus 10 is fully reversible even after foreshortening has been initiated and has advanced to the point illustrated in Figure 4C.

In Figure 4D, continued foreshortening causes male interlocking elements 44 of locks 40 to engage female interlocking elements 42. The male elements mate with the female elements, thereby locking apparatus 10 in the foreshortened configuration, as seen in Figure 4E. Actuators 50 are then pulled through eyelets 45 of male elements 44 to remove the actuators from apparatus 10, and actuators 62 are pulled through the proximal end of anchor 30 to uncouple actuators 60 from the apparatus, thereby separating delivery system 100 from apparatus 10. Fully deployed apparatus 10 is shown in Figure 4F.

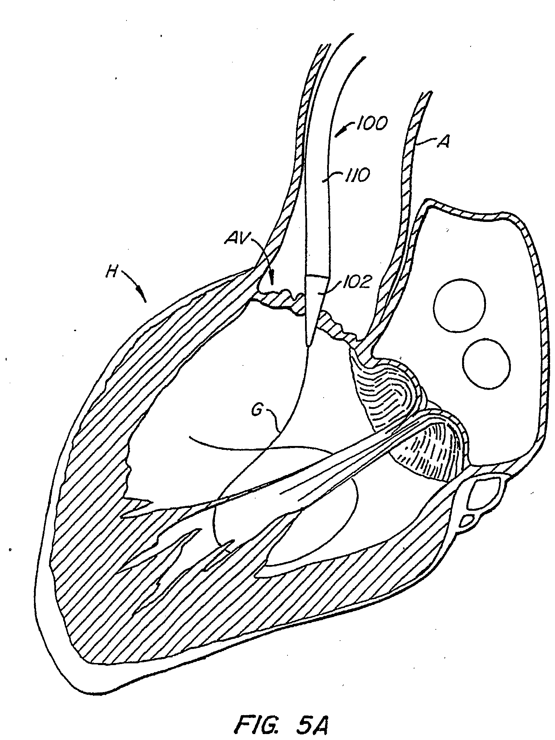

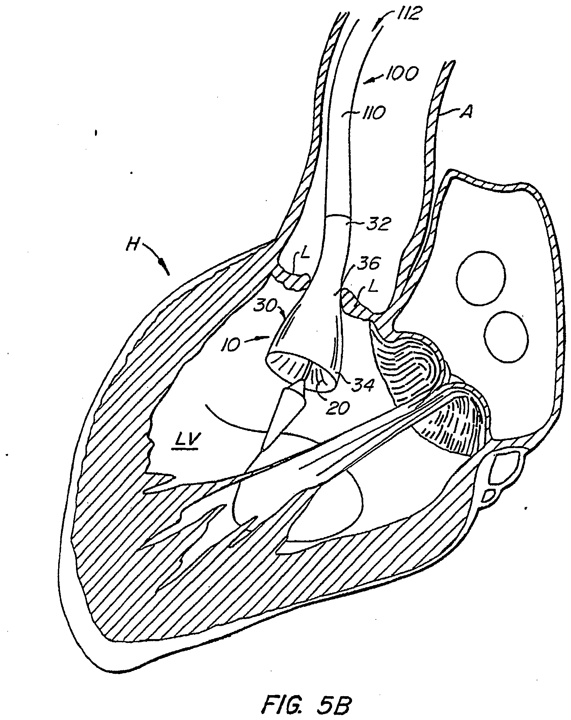

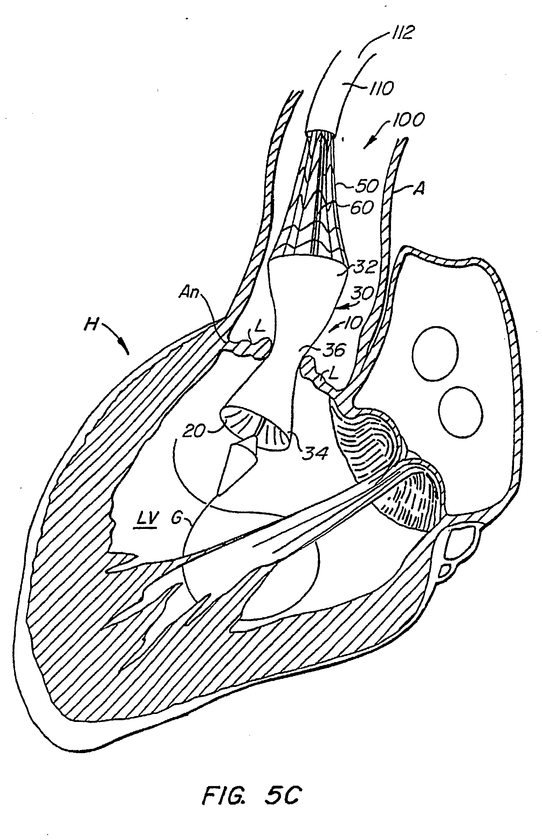

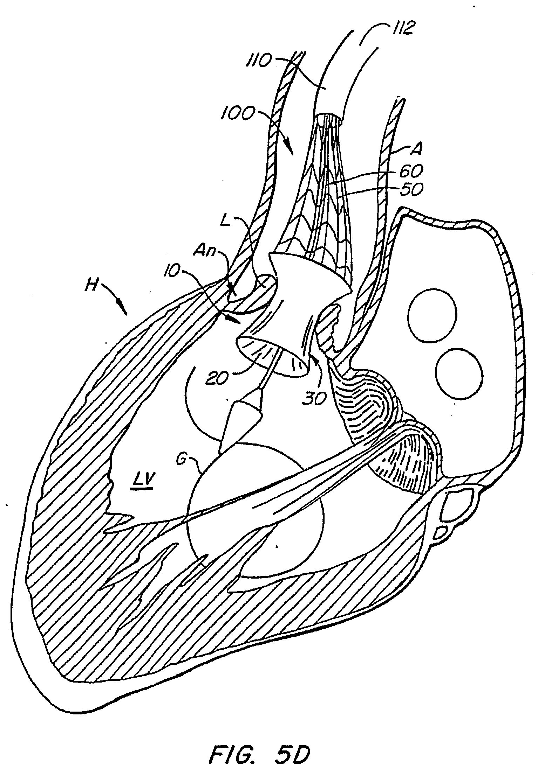

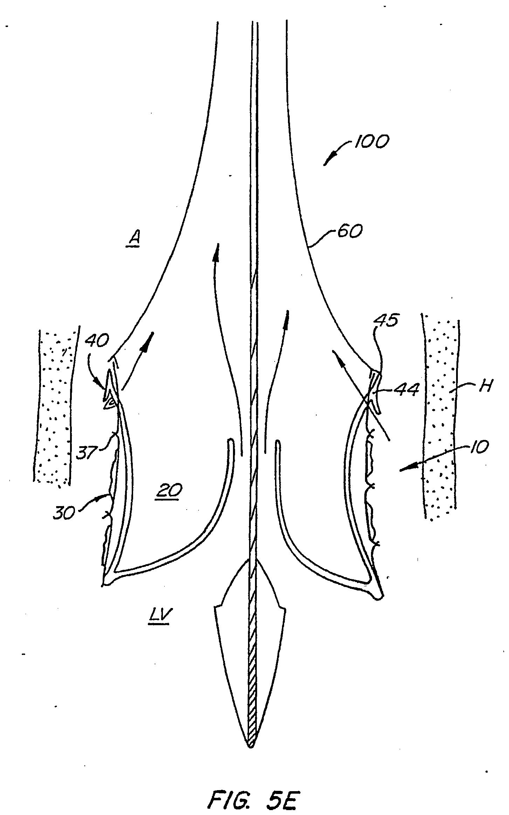

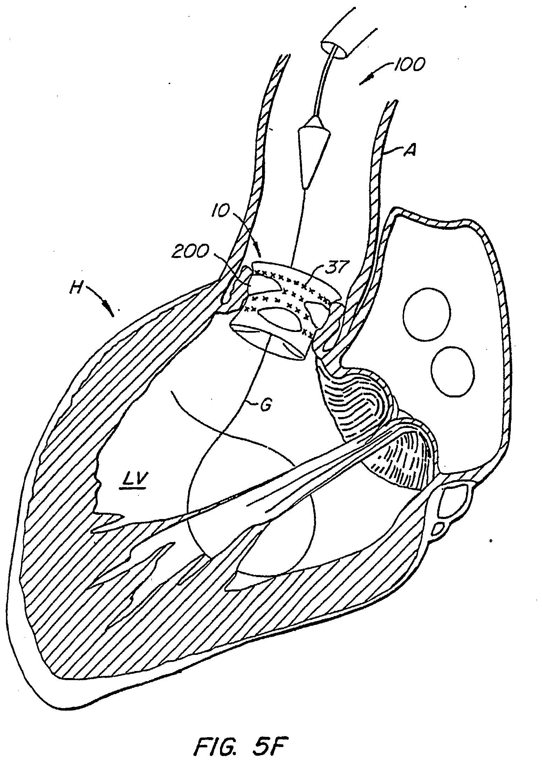

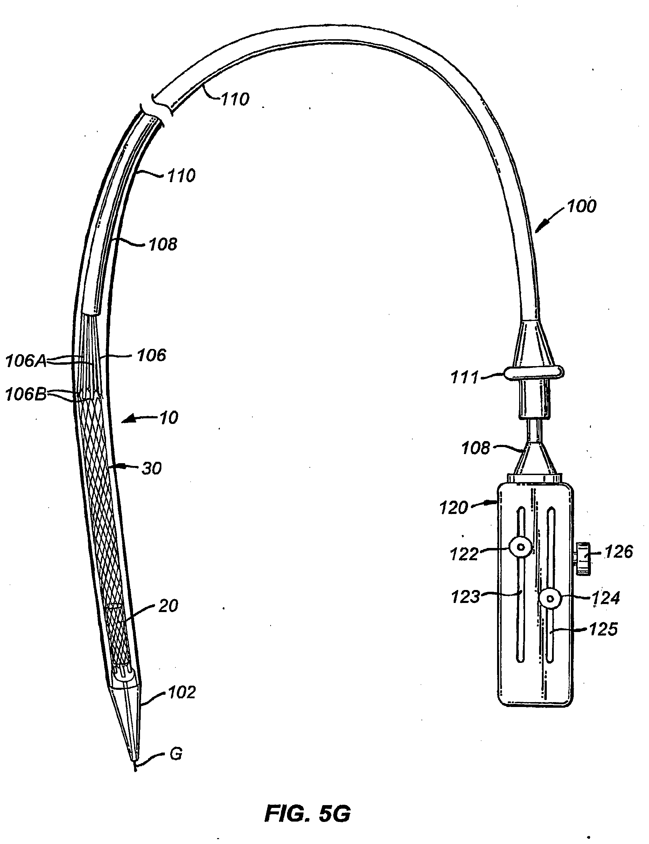

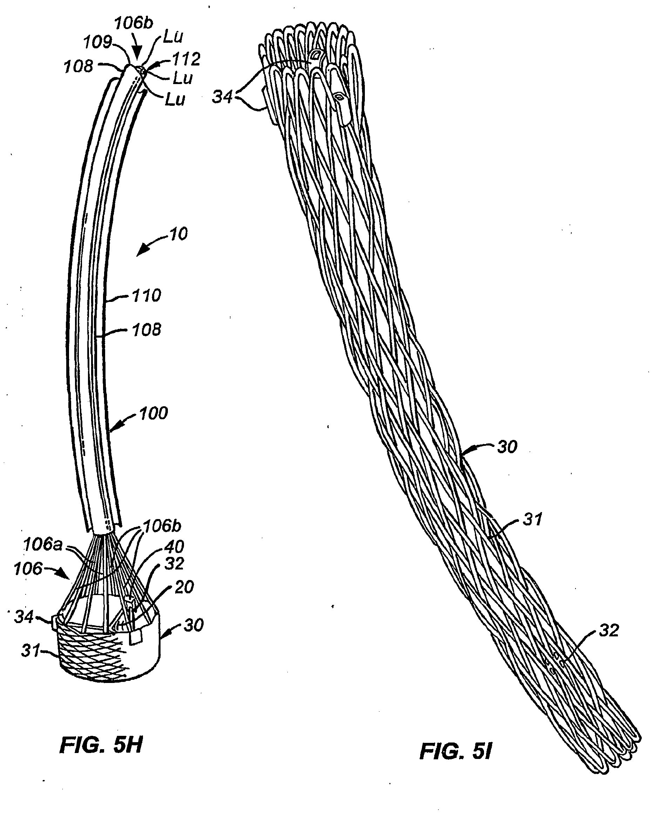

Referring to Figures 5, a method of percutaneously replacing a patient's diseased aortic valve with apparatus 10 and delivery system 100 is described. As seen in Figure 5A, sheath 110 of delivery system 100, having apparatus 10 disposed therein, is percutaneously advanced over guide wire G, preferably in a retrograde fashion (although an antegrade or hybrid approach alternatively may be used), through a patient's aorta A to the patient's diseased aortic valve AV. A nosecone 102 precedes sheath 110 in a known manner. In Figure 5B, sheath 110 is positioned such that its distal region is disposed within left ventricle LV of the patient's heart H.

Apparatus 10 is deployed from lumen 112 of sheath 110, for example, under fluoroscopic guidance, such that anchor 30 of apparatus 10 dynamically self-expands to a partially deployed configuration, as in Figure 5C. Advantageously, apparatus 10 may be retracted within lumen 112 of sheath 110 via actuators 50 - even after anchor 30 has dynamically expanded to the partially deployed configuration, for example, to abort the procedure or to reposition apparatus 10 or delivery system 100. As yet another advantage, apparatus 10 may be dynamically repositioned, e.g. via sheath 110 and/or actuators 60, in order to properly align the apparatus relative to anatomical landmarks, such as the patient's coronary ostia or the patient's native valve leaflets L. When properly aligned, skirt region 34 of anchor 30 preferably is disposed distal of the leaflets, while body region 36 is disposed across the leaflets and lip region 32 is disposed proximal of the leaflets.

Once properly aligned, actuators 50 are retracted relative to actuators 60 to impose foreshortening upon anchor 30 and expand apparatus 10 to the fully deployed configuration, as in Figure 5D. Foreshortening increases the radial strength of anchor 30 to ensure prolonged patency of valve annulus An, as well as to provide a better seal for apparatus 10 that reduces paravalvular regurgitation. As seen in Figure 5E, locks 40 maintain imposed foreshortening. Replacement valve 20 is properly seated within anchor 30, and normal blood flow between left ventricle LV and aorta A is thereafter regulated by apparatus 10. Deployment of apparatus 10 advantageously is fully reversible until locks 40 have been actuated.

As seen in Figure 5F, actuators 50 have been pulled from eyelets 45 of male elements 44 of locks 40, actuators 60 are decoupled from anchor 30, e.g. via actuators 62, and delivery system 100 is removed from the patient, thereby completing deployment of apparatus 10. Optional barb elements 37 engage the patient's native valve leaflets, e.g. to further preclude migration of the apparatus and/or reduce paravalvular regurgitation.

Figures 5G and 5H show further details of deployment using a deployment apparatus. Apparatus 10 is deployed from lumen Lu of sheath 110, for example, under fluoroscopic guidance by proximally retracting proximal handle 111 of sheath 110 relative to shaft 108, such that anchor 30 of apparatus 10 dynamically self-expands to the partially deployed configuration of Figure 5C. Advantageously, apparatus 10 may be retracted within lumen Lu of sheath 110 by retracting shaft 108 relative to the sheath, and thereby retracting actuators 106a coupled to anchor 30 relative to sheath 110. In this manner, anchor 30 may be retrieved even after the anchor has dynamically expanded to the partially deployed configuration, for example, to abort the procedure or to reposition apparatus 10 or delivery system 100. As yet another advantage, apparatus 10 may be dynamically repositioned, in order to properly align the apparatus relative to anatomical landmarks, such as the patient's coronary ostia or the patient's native valve leaflets L. When properly aligned, a distal region of anchor 30 preferably is disposed distal of the leaflets, while a central region of the anchor is disposed across the leaflets and a proximal region is disposed proximal of the leaflets.

Once properly aligned, actuators 106b are proximally retracted relative to actuators 106a, e.g., via knob 126 of handle 120, to impose foreshortening upon anchor 30 and further expand apparatus 10 to the fully deployed configuration, as in Figure 5D. Foreshortening increases the radial strength of anchor 30 to ensure prolonged patency of valve annulus An, as well as to provide a better seal for apparatus 10 that reduces paravalvular regurgitation. Lock 40 formed by engaging post lock elements 44 of posts 32 with anchor lock elements 34 of anchor 30 maintains imposed foreshortening. Replacement valve 20 is properly seated within anchor 30, and normal blood flow between left ventricle LV and aorta A is thereafter completely regulated by apparatus 10, although valve 20 is functional during deployment as well. Deployment of apparatus 10 advantageously is fully reversible until the locks have been actuated. Releasable lock prevention mechanisms may be provided to ensure that the locks are not actuated prematurely. Furthermore, the locks may be reversible, such that apparatus 10 may be retrieved or repositioned even after actuation of the locks.

Once apparatus 10 is fully expanded and locked in the expanded configuration, actuators 106a are decoupled from anchor 30 by actuating releasable attachment mechanisms, e.g., by retracting release actuators 112 relative to the actuators 106a via knob 122 of handle 120. Likewise, actuators 106b are decoupled from posts 32 by actuating releasable attachment mechanisms, e.g., by retracting release actuators 112 relative to the actuators 106b via knob 124 of handle 120. As seen in Figure 5E, delivery system 100 then may be removed from the patient, thereby completing deployment of apparatus 10. Optional barb elements 37 engage the patient's native valve leaflets, e.g. to preclude migration of the apparatus and/or to reduce paravalvular regurgitation.

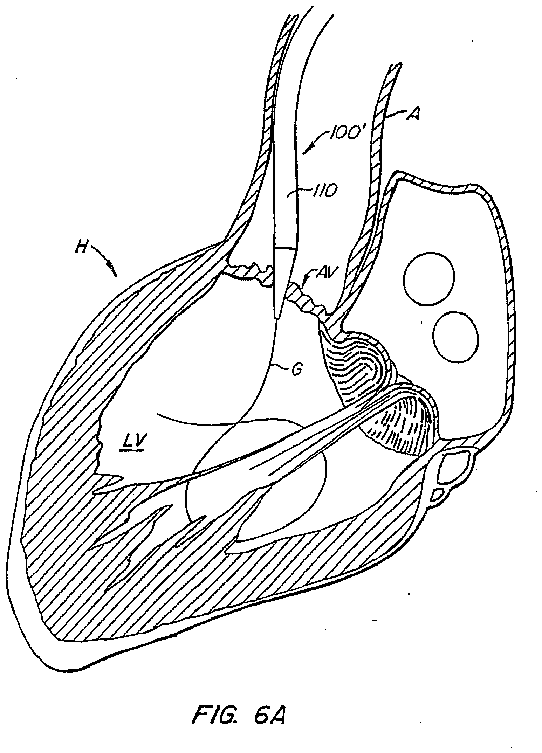

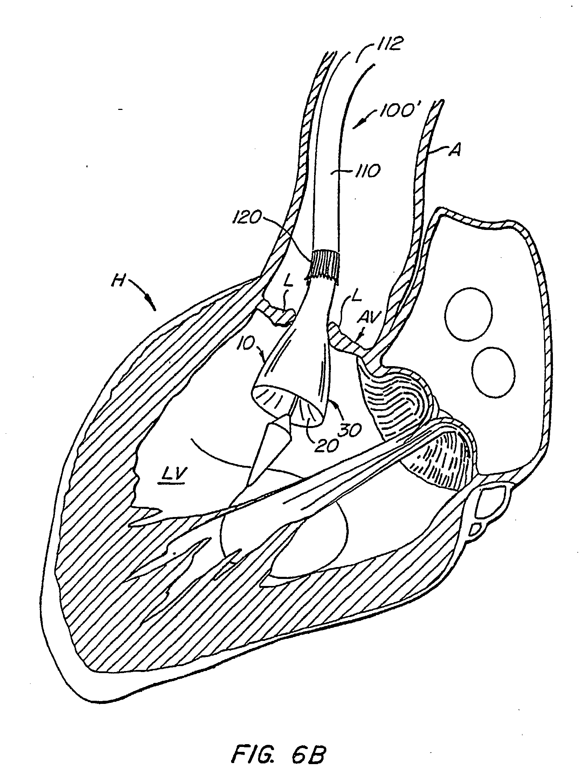

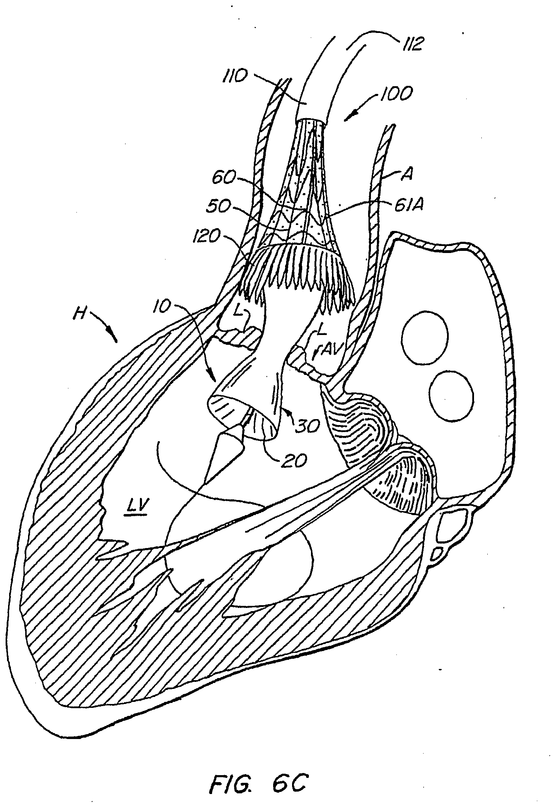

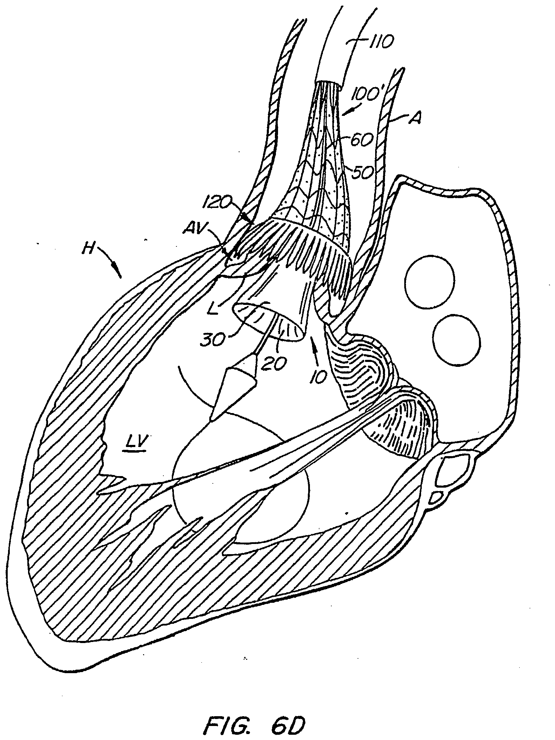

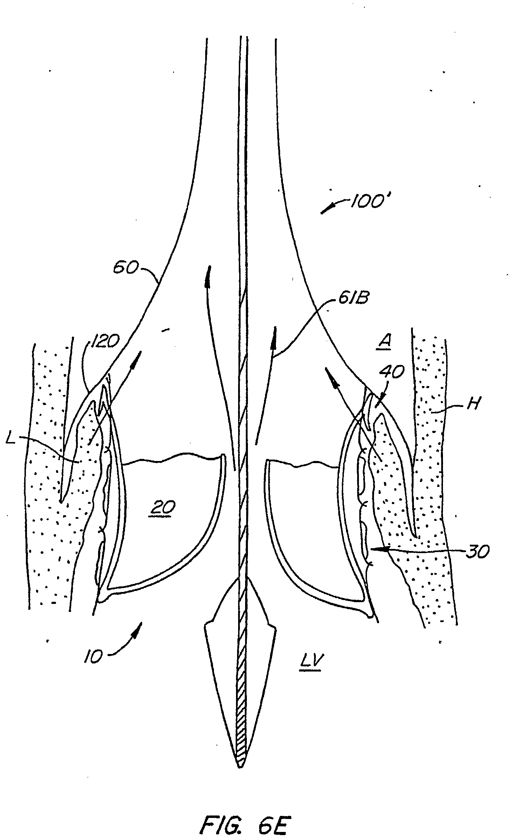

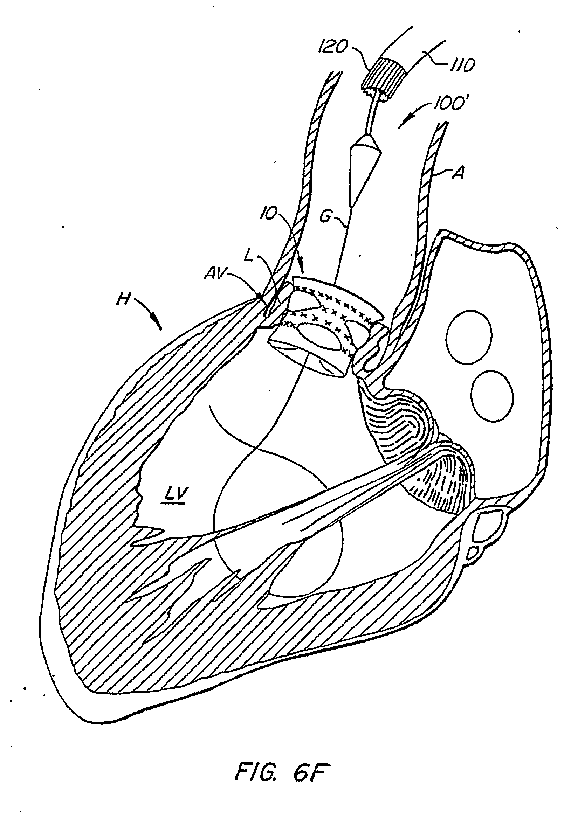

With reference now to Figures 6, a method of percutaneously replacing a patient's diseased aortic valve with apparatus 10 is provided, wherein proper positioning of the apparatus is ensured via positive registration of a modified delivery system to the patient's native valve leaflets. In Figure 6A, modified delivery system 100' delivers apparatus 10 to diseased aortic valve AV within sheath 110. As seen in Figures 6B and 6C, apparatus 10 is deployed from lumen 112 of sheath 110, for example, under fluoroscopic guidance, such that anchor 30 of apparatus 10 dynamically self-expands to a partially deployed configuration. As when deployed via delivery system 100, deployment of apparatus 10 via delivery system 100' is fully reversible until locks 40 have been actuated.

Delivery system 100' comprises leaflet engagement element 120, which preferably self-expands along with anchor 30. Engagement element 120 is disposed between actuators 60 of delivery system 100' and lip region 32 of anchor 30. Element 120 releasably engages the anchor. As seen in Figure 6C, the element is initially deployed proximal of the patient's native valve leaflets L. Apparatus 10 arid element 120 then may be advanced/dynamically repositioned until engagement element positively registers against the leaflets, thereby ensuring proper positioning of apparatus 10. Also delivery system 100' includes filter structure 61A (e.g., filter membrane or braid) as part of push actuators 60 to act as an embolic protection element. Emboli can be generated during manipulation and placement of anchor from either diseased native leaflet or surrounding aortic tissue and can cause blockage.
Arrows 61B in Figure 6E show blood flow through filter structure 61A where blood is allowed to flow but emboli is trapped in the delivery system and removed with it at the end of the procedure.

Alternatively, foreshortening may be imposed upon anchor 30 while element 120 is disposed proximal of the leaflets, as in Figure 6D. Upon positive registration of element 120 against leaflets L, element 120 precludes further distal migration of apparatus 10 during additional foreshortening, thereby reducing a risk of improperly positioning the apparatus. Figure 6E details engagement of element 120 against the native leaflets. As seen in Figure 6F, once apparatus 10 is fully deployed, element 120, actuators 50 and actuators 60 are decoupled from the apparatus, and delivery system 100' is removed from the patient, thereby completing the procedure.

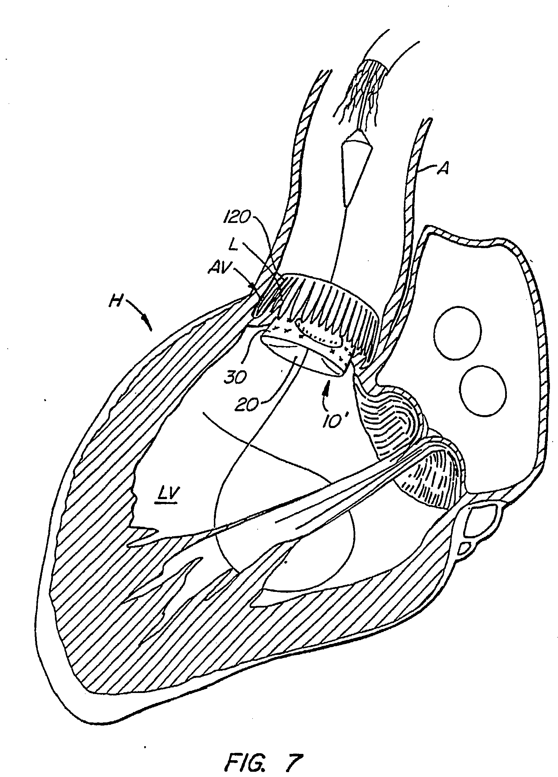

With reference to Figure 7, an alternative embodiment of the apparatus of Figures 6 is described, wherein leaflet engagement element 120 is coupled to anchor 30 of apparatus 10', rather than to delivery system 100. Engagement element 120 remains implanted in the patient post-deployment of apparatus 10'. Leaflets L are sandwiched between lip region 32 of anchor 30 and element 120 in the fully deployed configuration. In this manner, element 120 positively registers apparatus 10' relative to the leaflets and precludes distal migration of the apparatus over time.

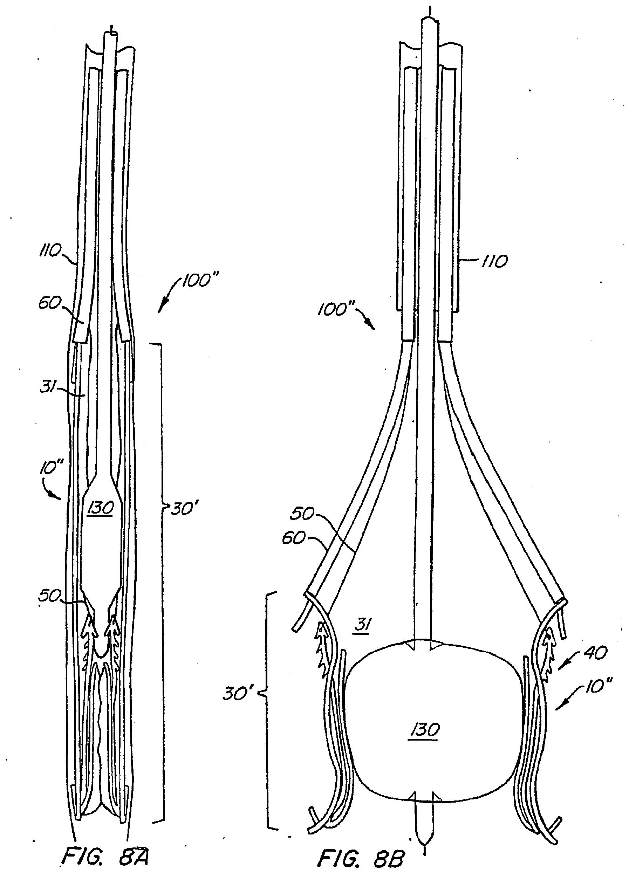

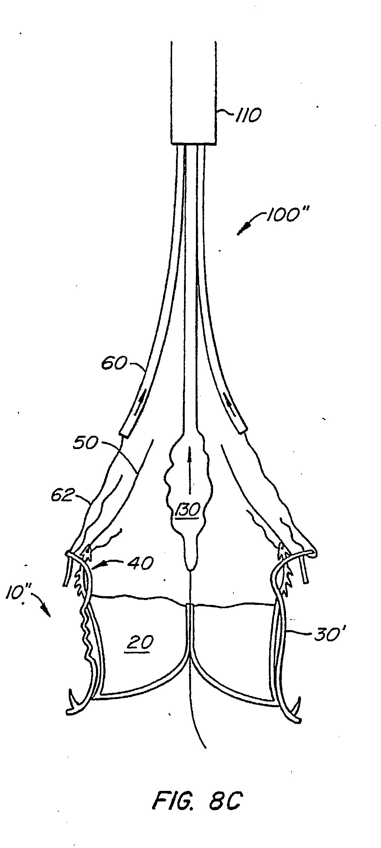

Referring now to Figures 8, an alternative delivery system adapted for use with a balloon expandable embodiment of the present invention is described. In Figure 8A, apparatus 10" comprises anchor 30' that maybe fabricated from balloon-expandable materials. Delivery system 100" comprises inflatable member 130 disposed in a deflated configuration within lumen 31 of anchor 30'. In Figure 8B, optional outer sheath 110 is retracted, and inflatable member 130 is inflated to expand anchor 30' to the fully deployed configuration. As inflatable member 130 is being deflated, as in earlier embodiments, actuators 50 and 62 and actuators 60 maybe used to assist deployment of anchor 30' and actuation of locks 40, as well as to provide reversibility and retrievability of apparatus 10" prior to actuation of locks 40. Next, actuators 50 and 62 and actuators 60 are removed from apparatus 10", and delivery system 100" is removed, as seen in Figure 8C.

As an alternative delivery method, anchor 30' may be partially deployed via partial expansion of inflatable member 130. The inflatable member would then be advanced within replacement valve 20 prior to inflation of inflatable member 130 and full deployment of apparatus 10". Inflation pressures used will range from about 3,04 to 6,08 bar [3 to 6 atm], or more preferably from about 4,05 to 5,07 bar [4 to 5 atm], though higher and lower bar [atm] pressures may also be used (e.g., greater than 3,04 bar [3 atm], more preferably greater than 4,05 bar [4 atm], more preferably greater than 5,07 bar [5 atm], or more preferably greater than 6,08 bar [6 atm]). Advantageously, separation of inflatable member 130 from replacement valve 20, until partial deployment of apparatus 10" at a treatment site, is expected to reduce a delivery profile of the apparatus, as compared to previously known apparatus. This profile reduction may facilitate retrograde delivery and deployment of apparatus 10", even when anchor 30' is balloon-expandable.

Although anchor 30' has illustratively been described as fabricated from balloon-expandable materials, it should be understood that anchor 30' alternatively may be fabricated from self-expanding materials whose expansion optionally may be balloon-assisted. In such a configuration, anchor 30' would expand to a partially deployed configuration upon removal of outer sheath 110. If required, inflatable member 130 then would be advanced within replacement valve 20 prior to inflation. Inflatable member 130 would assist full deployment of apparatus 10", for example, when the radial force required to overcome resistance from impinging tissue were too great to be overcome simply by manipulation of actuators 50 and actuators 60. Advantageously, optional placement of inflatable member 130 within replacement valve 20, only after dynamic self-expansion of apparatus 10" to the partially deployed configuration at a treatment site, is expected to reduce a delivery profile of the apparatus, as compared to previously known apparatus. This reduction may facilitate retrograde delivery and deployment of apparatus 10".

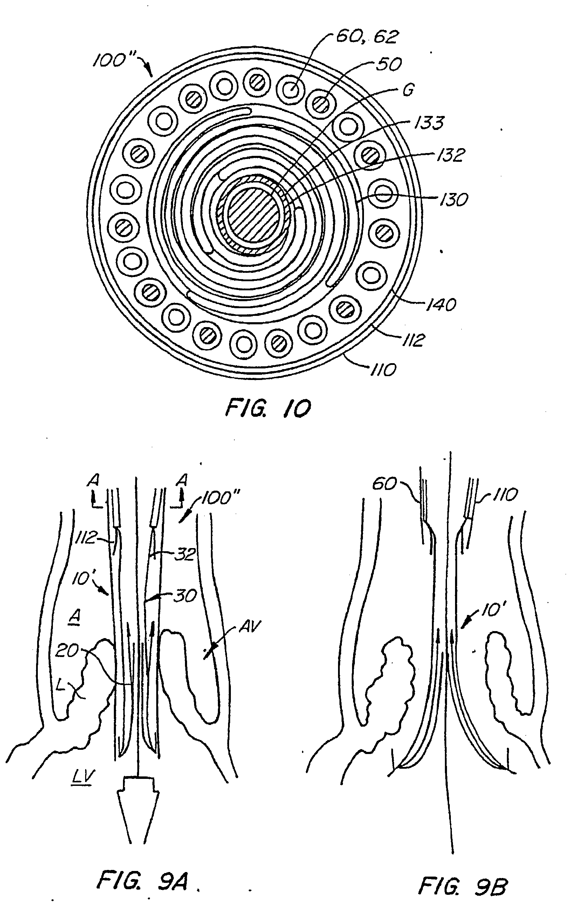

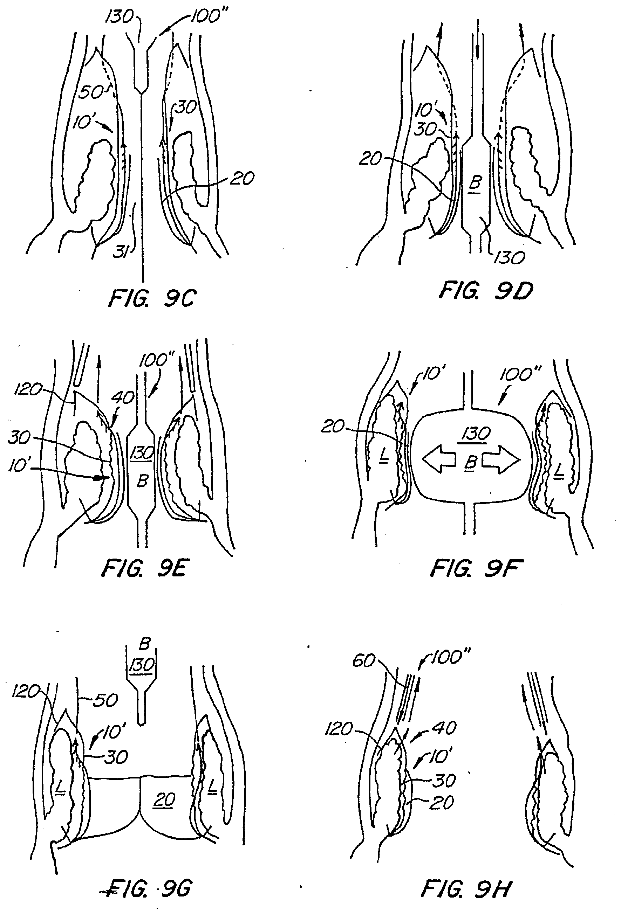

With reference to Figures 9 and 10, methods and apparatus for a balloon-assisted embodiment of the present invention are described in greater detail. Figures 9 and 10 illustratively show apparatus 10' of Figures 7 used in combination with delivery system 100" of Figures 8. Figure 10 illustrates a sectional view of delivery system 100". Inner shaft 132 of inflatable member 130 preferably is about 4 Fr in diameter, and comprises lumen 133 configured for passage of guidewire G, having a diameter of about 8.89 mm [0.035"], therethrough. Push actuators 60 and pull actuators 50 pass through guidetube 140, which preferably has a diameter of about 15 Fr or smaller. Guide tube 140 is disposed within lumen 112 of outer sheath 110, which preferably has a diameter of about 17 Fr or smaller.

In Figure 9A, apparatus 10' is delivered to diseased aortic valve AV within lumen 112 of sheath 110. In Figure 9B, sheath 110 is retracted relative to apparatus 10' to dynamically self-expand the apparatus to the partially deployed configuration. Also retracted and removed is nosecone 102 which is attached to a pre-slit lumen (not shown) that facilitates its removal prior to loading and advancing of a regular angioplasty balloon catheter over guidewire and inside delivery system 110.

In Figure 9C, pull actuators 50 and push actuators 60 are manipulated from external to the patient to foreshorten anchor 30 and sufficiently expand lumen 31 of the anchor to facilitate advancement of inflatable member 130 within replacement valve 20. Also shown is the tip of an angioplasty catheter 130 being advanced through delivery system 110.

The angioplasty balloon catheter or inflatable member 130 then is advanced within the replacement valve, as in Figure 9D, and additional foreshortening is imposed upon anchor 30 to actuate locks 40, as in Figure 9E. The inflatable member is inflated to further displace the patient's native valve leaflets L and ensure adequate blood flow through, and long-term patency of, replacement valve 20, as in Figure 9F. Inflatable member 130 then is deflated and removed from the patient, as in Figure 9G. A different size angioplasty balloon catheter could be used repeat the same step if deemed necessary by the user. Push actuators 60 optionally may be used to further set leaflet engagement element 120, or optional barbs B along posts 38, more deeply within leaflets L, as in Figure 9H. Then, delivery system 100" is removed from the patient, thereby completing percutaneous heart valve replacement.

As will be apparent to those of skill in the art, the order of imposed foreshortening and balloon expansion described in Figures 9 and 10 is only provided for the sake of illustration. The actual order may vary according to the needs of a given patient and/or the preferences of a given medical practitioner. Furthermore, balloon-assist may not be required in all instances, and the inflatable member may act merely as a safety precaution employed selectively in challenging clinical cases.

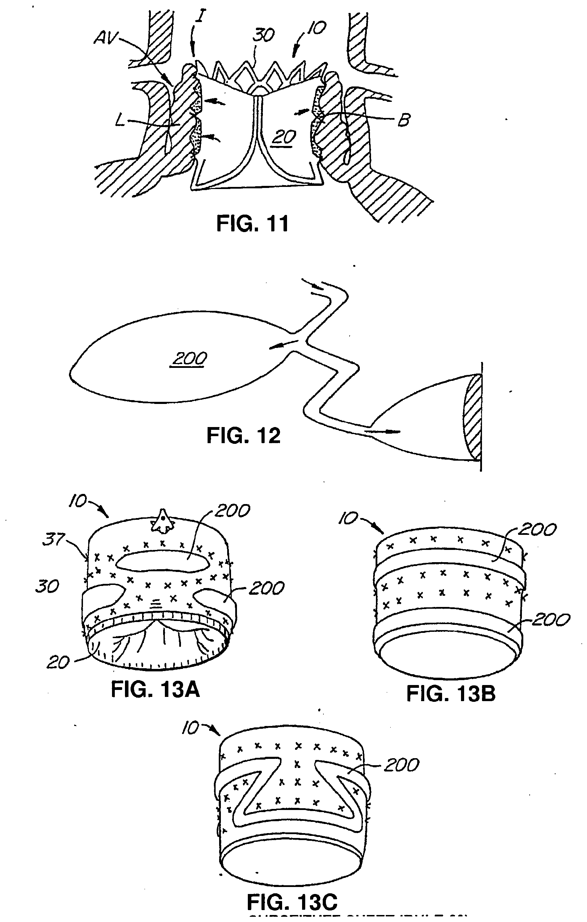

With reference now to Figure 11, a risk of paravalvular leakage or regurgitation around apparatus of the present invention is described. In Figure 11, apparatus 10 has been implanted at the site of diseased aortic valve AV, for example, using techniques described hereinabove. The surface of native valve leaflets L is irregular, and interface I between leaflets L and anchor 30 may comprise gaps where blood B may seep through. Such leakage poses a risk of blood clot formation or insufficient blood flow.

Referring to Figure 12, optional elements for reducing regurgitation or leakage are described. Compliant sacs 200 may be disposed about the exterior of anchor 30 to provide a more efficient seal along irregular interface I. Sacs 200 may be filled with an appropriate material, for example, water, blood, foam or a hydrogel. Alternative fill materials will be apparent.

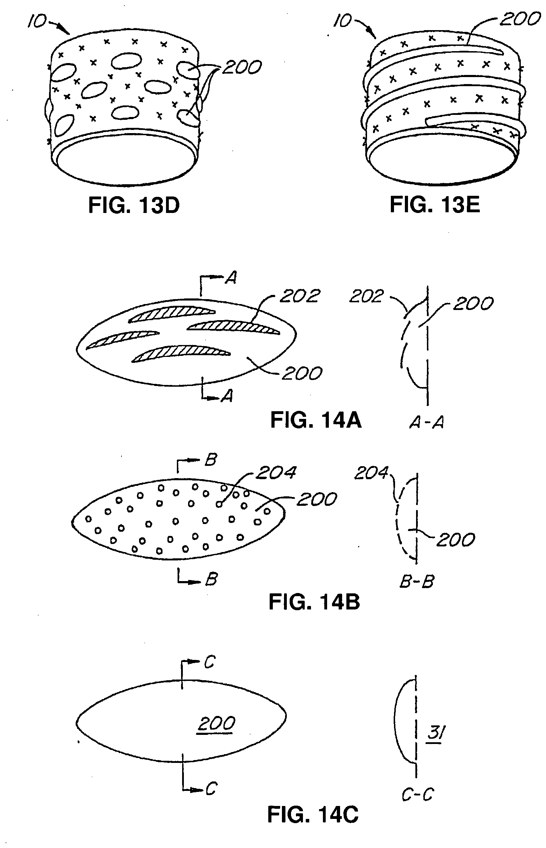

With reference to Figures 13, illustrative arrangements for sacs 200 are provided. In Figure 13A, sacs 200 are provided as discrete sacs at different positions along the height of anchor 30. In Figure 13B, the sacs are provided as continuous cylinders at various heights. In Figure 13C, a single sac is provided with a cylindrical shape that spans multiple heights. The sacs of Figure 13D are discrete, smaller and provided in larger quantities. Figure 13E provides a spiral sac. Alternative sac configurations will be apparent to those of skill in the art.

With reference to Figures 14, exemplary techniques for fabricating sacs 200 are provided. In Figure 14A, sacs 20 comprise 'fish-scale' slots 202 that may be back-filled, for example, with ambient blood passing through replacement valve 20. In Figure 14B, the sacs comprise pores 204 that may be used to fill the sacs. In Figure 14C, the sacs open to lumen 31 of anchor 30 and are filled by blood washing past the sacs as the blood moves through apparatus 10.

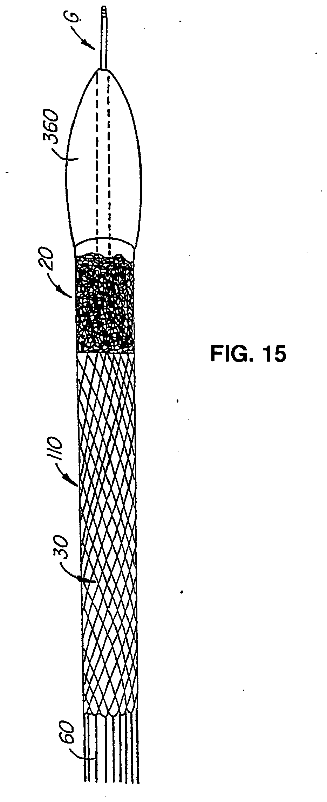

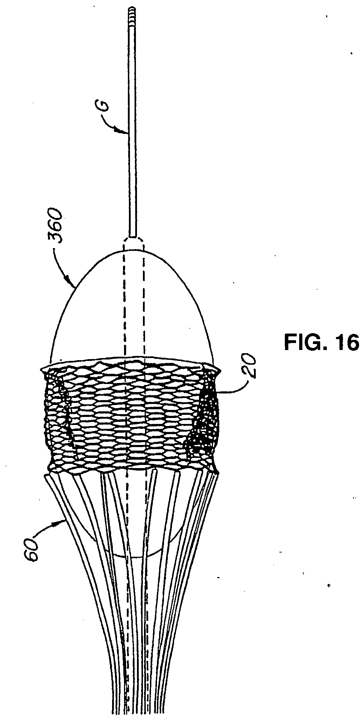

Figures 15 and 16 show yet another embodiment of the delivery and deployment apparatus of the invention. As an alternative to the balloon expansion method described with respect to Figures 8, in this embodiment the nosecone (e.g., element 102 of Figures 5) is replaced by an angioplasty balloon catheter 360. Thus, expandable balloon cathether 360 precedes sheath 110 on guidewire G. When anchor 30 and valve 20 are expanded through the operation of actuators 60 and the control actuators (not shown) as described above, balloon cathether 360 is retracted proximally within the expanded anchor and valve and expanded further as described above with respect to Figures 8.

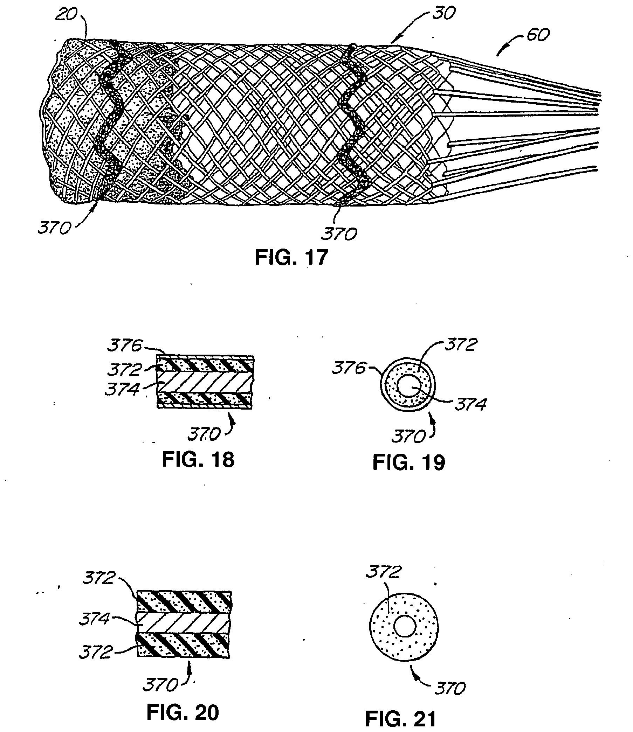

Figures 17-21 show seals 370 that expand over time to seal the interface between the anchor and valve and the patient's tissue. Seals 370 are preferably formed from Nitinol wire surrounded by an expandable foam. As shown in cross-section in Figures 18 and 19, at the time of deployment, the foam 372 is compressed about the wire 374 and held in the compressed form by a time-released coating 376. After deployment, coating 376 dissolves in vivo to allow foam 372 to expand, as shown in Figures 20 and 21.

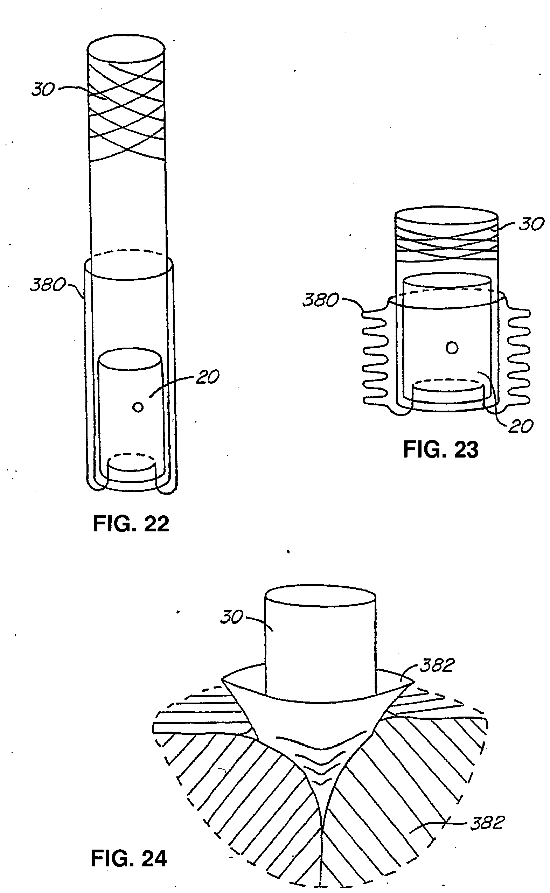

Figures 22-24 show another way to seal the replacement valve against leakage. A fabric seal 380 extends from the distal end of valve 20 and back proximally over anchor 30 during delivery. When deployed, as shown in Figures 23 and 24, fabric seal 380 bunches up to create fabric flaps and pockets that extend into spaces formed by the native valve leaflets 382, particularly when the pockets are filled with blood in response to backflow blood pressure. This arrangement creates a seal around the replacement valve.

Figures 25A and 25B illustrate one embodiment of a delivery system/deployment tool and apparatus in accordance with the present invention. As seen in Figure 25A, apparatus 10 may be collapsed for delivery within delivery system/deployment tool 100. Delivery system 100 includes guidewire G, nosecone 102, anchor actuation elements 106, multi-lumen shaft or catheter 108 having optional central lumen 109 and a plurality of circumferentially disposed lumens Lu, external sheath 110 having optional proximal handle 111, and confrol handle 120. Nosecone 102 may, for example, be manipulated via a shaft extending through central lumen 109 of multi-lumen catheter 108.

Anchor actuation elements 106 preferably comprise both proximal anchor actuation elements and distal anchor actuation elements. The proximal anchor actuation elements may, for example, comprise actuators 106a that are releasably coupled to a proximal region of anchor 30 of apparatus 10 via releasable attachment mechanisms for manipulating a proximal region of apparatus 10. The distal anchor actuation elements may comprise actuators 106b that are releasably coupled to a distal region of anchor 30 via releasable attachment mechanisms for manipulating the distal region of apparatus 10. In some embodiments, the distal anchor actuation elements may comprise posts or anchor attachment elements 32 of anchor 30 and the releasable attachment mechanisms connecting actuators 106b to posts 32. In an alternative configuration, the proximal anchor actuation elements may be releasably coupled to a proximal region of apparatus 10 through posts and releasable attachment mechanisms for manipulation of a proximal region of the apparatus, while the distal anchor actuation elements may connect to a distal region of anchor 30 via releasable attachment mechanisms to manipulate a distal region of the apparatus. As another alternative, both proximal and distal anchor actuation element may connect to anchor 30 via releasable attachment mechanisms.

In the embodiment shown in Figures 25, actuators 106a may, for example, include stiff finger elements extending from a distal region of multi-lumen shaft 108, while actuators 106b may include control elements (e.g., stands of suture, or metal or polymer wires) which pass through one or more lumens Lu of shaft 108. Release actuators 112 for the releasable attachment mechanisms for both sets of actuators also may pass through one or more lumens Lu of shaft 108. The release actuators may comprise, for example, control elements (e.g., strands of suture, or metal or polymer wires), covers, mandrels, elongated elements, friction surfaces, wrap portions, interference shapes, etc. The release actuators preferably are movable relative to anchor actuation elements 106, e.g., via control handle 120.

Control handle 120 is coupled to multi-lumen shaft 108. Knob 122 disposed in slot 123 may actuate release actuators 112 that couple actuators 106a of anchor actuation elements 106 to apparatus 10. Likewise, knob 124 disposed in slot 125 may actuate release actuators 112 that couple actuators 106b of anchor actuation elements 106 to posts 32 of anchor 30 of apparatus 10. Handle 120 also comprises knob 126 for, e.g., manipulating the actuators 106b to control movement of the distal region of apparatus 10 relative to its proximal region. Conversely, controlled movement of the proximal region of apparatus 10 relative to its distal region may be achieved by holding knob 126 stationary while advancing or retracting handle 120. Knob 126 optionally may move actuators 106b in unison with their concomitant release actuators 112.

Apparatus 10 comprises anchor 30 and replacement valve 20. Anchor 30 preferably comprises a braid. Such braid can have closed ends at either or both its ends. Replacement valve 20 is preferably coupled to the anchor along posts 32, e.g., along a valve attachment structure, such as a tab and/or a plurality of holes. Posts 32, therefore, may function as valve supports and may be adapted to support the replacement valve within the anchor, h the embodiment shown, there are three posts, corresponding to the valve's three commissural attachment points. The posts can be attached to the braid portion of anchor 30. The posts can be attached to the braid's distal end, as shown in Figure 26A, central region, or proximal end. Replacement valve 20 can be composed of a synthetic material and/or may be derived from animal tissue. Replacement valve 20 is preferably configured to be secured within anchor 30.

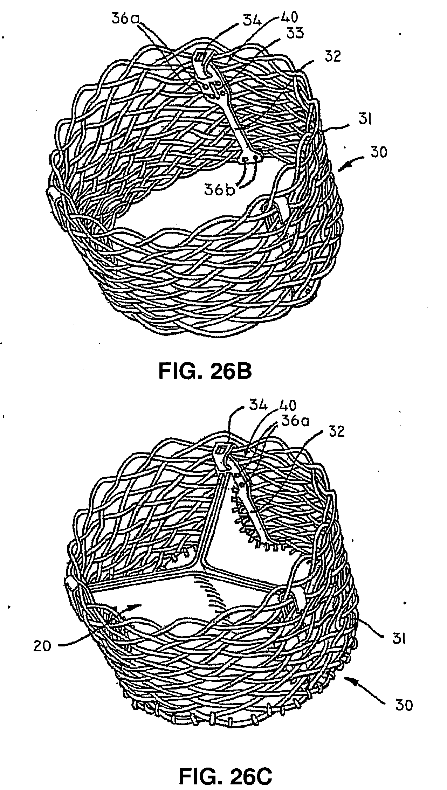

Anchor 30 comprises a plurality of anchor lock elements 34, e.g., buckles 34, attached to its proximal region, one for each post 32. Posts 32 may comprise a lock element that forms a two-part locking mechanism with anchor lock elements 34 for maintaining anchor 30 in a deployed or expanded configuration (e.g., as illustrated in Figures 25B, 26B and 26C).

In this embodiment, anchor 30 is formed from a collapsible and expandable wire braid. Anchor braid 30 is preferably self-expanding and is preferably formed from a material such as Nitinol, cobalt-chromium steel or stainless steel wire using one or more strands of wire. Delivery and deployment of braided anchor 30 is similar to the delivery and deployment of the anchors described in U.S. Patent Appl. Ser. No. 10/746,120. Specifically, in one embodiment described below, during deployment braided anchor 30 is actively foreshortened by proximally retracting the actuators 106b relative to the actuators 106a to expand and lock the anchor in place. In some embodiments, foreshortening may expand anchor 30 to a radially symmetrical, bilaterally symmetrical, or asymmetrical expanded shape. The foreshortening step can include expanding a first region of the anchor to a first diameter and a second region of the anchor to a second diameter larger than the first diameter. A third region may also be expanded to a diameter larger than the first diameter. The expansion of various regions of the anchor (e.g., the distal region) can be especially useful in locating the aortic valve and centering the anchor within it. Preferably, the secured anchor does not interfere with the mitral valve or the ostia. In some embodiments, the anchor is allowed to self-expand prior to the foreshortening step.

As seen in Figures 25, after endovascular delivery through sheath 110 to the vicinity of the patient's native valve (such as the aortic valve), apparatus 10 may be expanded from the collapsed delivery configuration of Figure 25A to the expanded deployed configuration of Figure 25B using delivery system/deployment tool 100. To deploy apparatus 10, external sheath 110 may be retracted relative to apparatus 10 by proximally retracting sheath handle 111 relative to control handle 120. Sheath 110 is thereby removed from the exterior of apparatus 10, permitting the anchor 30 to self-expand. For example, if anchor braid 30 is composed of a shape memory material, it may self-expand to or toward its "at-rest" configuration. This at-rest configuration of the braid can be, for example its expanded configuration, a collapsed configuration, or a partially expanded configuration between the collapsed configuration and the expanded configuration, or some combination. In preferred embodiments, the anchor's at-rest configuration is between the collapsed configuration and the expanded configuration. Depending on the at-rest diameter of the braid and the diameter of the patient's anatomy at the chosen deployment location, the anchor may or may not self-expand to come into contact with the diameter of the patient's anatomy at that location.

In its collapsed configuration, anchor 30 preferably has a collapsed delivery diameter between about 3 to 30 Fr, or more preferably 6 to 28 Fr, or more preferably 12 to 24 Fr. In some embodiments, anchor 30 in its collapsed configuration will have a length ranging from about 5 to about 170 mm, more preferably from about 10 to about 160 mm, more preferably from about 15 to about 150 mm, more preferably from about 20 to about 140 mm, or more preferably from about 25 mm to about 130 mm.

Similarly, in its expanded configuration, anchor 30 preferable has a diameter ranging between about 10 to about 36 mm, or more preferably from about 24 to about 33 mm, or more preferably from about 24 to about 30 mm. In some embodiments, anchor 30 in its expanded configuration will have a length ranging from about 1 to about 50 mm, more preferably from about 2 to about 40 mm, more preferably from about 5 to about 30 mm, or more preferably from about 7 to about 20 mm.

Overall, the ratio of deployed to collapsed/sheathed lengths is preferably between about 0.05 and 0.5, more preferably about 0.1 to 0.35, or more preferably about 0.15 to 0.25. In any of the embodiments herein, anchor 30 in its expanded configuration preferably has a radial crush strength that maintains the anchor substantially un-deformed in response to a pressure of up to about 0,51 bar [0.5 atm] directed substantially radially inward toward the central axis, or more preferably up to about 2,03 bar [2 atm] directed substantially radially inward toward the central axis, in addition, in any of the embodiments herein, the anchor preferably has an axial spring constant of between about 10 to 250 g/cm, more preferably between about 20 to 200 g/cm, or more preferably between about 40 to 160 g/cm. In addition, in any of the embodiments herein, the anchor is preferably adapted to support the replacement valve at the anchor site in response to a differential pressure of up to about 120 mm Hg, more preferably up to about 240 mm Hg, or more preferably up to about 320 mm Hg.

As seen in Figure 25B, anchor 30 may be expanded to a fully deployed configuration from a partial deployed configuration (e.g., self-expanded configuration) by actively foreshortening anchor 30 during endovascular deployment. In some embodiments, foreshortening of the apparatus involves applying a distally directed force on the proximal end of the anchor by one or more anchor actuation elements to move the proximal end of the anchor distally while maintaining the position of the distal end of the anchor. For example, the proximal region of anchor 30 may be pushed distally by certain anchor actuation elements 106, e.g., actuators 106a. Alternatively, foreshortening of the apparatus involves applying a proximally directed force on the distal end of the anchor by one or more anchor actuation elements to move the distal end of the anchor proximally while maintaining the position of the proximal end of the anchor. For example, the distal region of anchor 30 may be pulled proximally via a proximally directed force applied by post actuation elements 106b, this force opposed by anchor actuators 106a.

Anchor actuation elements 106 preferably are adapted to expand radially as the anchor expands radially and to contract radially as the anchor contracts radially. Furthermore, proximally or distally directed forces by the anchor actuation elements on one end of the anchor do not diametrically constrain the opposite end of the anchor. In addition, when a proximally or distally directed force is applied on the anchor by the anchor actuation elements, it is preferably applied without passing any portion of a deployment system through a center opening of the replacement valve. This arrangement enables the replacement valve to operate during deployment and before removal of the deployment system.

The distal anchor actuation elements may include, for example, actuators 106b and/or release actuators 112 that are controlled, e.g., by control knobs 124 and 126 of confrol handle 120. Similarly, the proximal regions of anchor 30 may be pushed distally via proximal anchor actuation elements, e.g., actuators 106a, at the proximal region of the anchor. The proximal anchor actuation elements facilitate application of a distally directed force to the proximal end of anchor 30 to move or constrain the proximal end of the anchor distally and are controlled through motion of shaft 108 relative to the distal anchor actuation elements. Control knob 122 of control handle 120 may control release actuators 112 for releasing the proximal anchor actuation elements from the braid. The proximal anchor actuation elements may be further adapted to expand as the proximal end of the anchor expands radially during application of a distally directed force on the proximal end of the anchor. Preferably, the proximal anchor actuation elements apply a distally directed force on the proximal end of the anchor system through a plurality of actuators 106a in order to expand the braid of anchor 30. Such braid expansion optionally may be assisted via inflation of a balloon catheter (see Figures 15 and 16) reversibly disposed within apparatus 10, as described in U.S. Patent Appl. Ser. No. 10/746,120.

In the fully deployed configuration, lock elements of posts 32 and anchor lock elements or buckles 34 of anchor 30 may be used to lock and maintain the anchor in the deployed configuration. Apparatus 10 may be repositioned or retrieved from the patient until the lock elements of posts 32 have been interlocked with anchor lock elements 34 of anchor 30 to form lock 40. In one embodiment, actuators 106b and attendant release actuators 112 comprise control elements attached to posts 32 that are threaded through buckles 34 so that the proximally directed force exerted on posts 32 by the control elements during deployment pulls a lock element of posts 32 toward and through buckles 34 to form lock 40. In this manner, the control elements may act as both anchor actuators and lock actuators.

Such lock optionally may be selectively reversible to allow for repositioning and/or retrieval of apparatus 10 during or post-deployment. When the lock is selectively reversible, the apparatus may be repositioned and or retrieved as desired, i.e., even after actuation of lock 40.

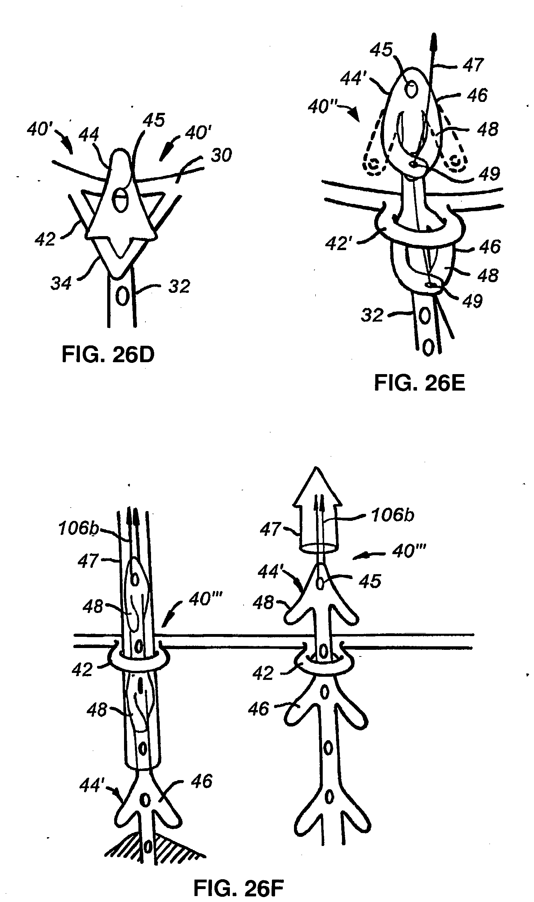

Locks used herein may also include a plurality of levels of locking wherein each level of locking results in a different amount of expansion. For example, the anchor lock elements at the proximal end of the post can have multiple configurations for locking within the buckle wherein each configuration results in a different amount of anchor expansion (see, e.g., Figure 26F). Such locking mechanisms may, for example, comprise ratchets having multiple lock locations. Furthermore, lock alignment features may be provided to facilitate alignment of the post and anchor lock elements, such as a hinge or an oversized width of the post or anchor lock elements. Furtherstill, lock prevention mechanisms may be provided to preclude locking until desired by a medical practitioner.

When apparatus 10 is placed across a patient's diseased heart valve, anchor 30 may be used to displace the patient's native valve leaflets, and replacement valve 20 will thereafter serve in place of the native valve. After final positioning and expansion, apparatus 10 may be decoupled from delivery system 100 by decoupling the proximal and distal anchor actuation elements 106 from the apparatus via releasable attachment mechanisms, e.g., by decoupling proximal actuators 106a from braided anchor 30 and distal actuators 106b from posts 32 of the anchor via the releasable attachment mechanisms. Moving release actuators 112, e.g., using knobs 122 and 124 of handle 120, may, for example, actuate the releasable attachment mechanisms. Preferably, the releasable attachment mechanisms may be actuated by moving the release actuator(s) less than about 2,54 cm [1 inch]. After decoupling, delivery system/deployment tool 100 may be removed from the patient, thereby completing endovascular replacement of a patient's heart valve.

Prior to implantation of replacement valve apparatus described herein, it may be desirable to perform a valvuloplasty on the patient's diseased valve by inserting a balloon into the valve and expanding it using, e.g., saline mixed with a contrast agent, in addition to preparing the valve site for implant, fluoroscopic viewing of the valvuloplasty will help determine the appropriate size of replacement valve implant to use.

Figures 26A-26C show further details of anchor 30 of apparatus 10. Figure 26A shows the apparatus in a collapsed configuration, such as for delivery within a sheath or other lumen or for retrieval and recapture into a sheath or other lumen. Figures 26B and 26C show the anchor and valve in an expanded and locked configuration.

As shown in Figure 26B, anchor 30 illustratively has three posts and three buckles. As seen in Figure 26C, the three leaflets of replacement valve 20 may be, coupled to the three posts 32 along valve support structures. Thus, posts 32 act as valve supports. The posts, unlike the braid, do not collapse or expand. In some embodiments, a post 32 has one or more proximal slots 33, at least one proximal hole 36a and at least one distal hole 36b. Leaflet tissue may, for example, be passed through slot 33 and sutured in place via suture routed through one or more proximal holes 36a. In this manner, slot(s) 33 and hole(s) 36a may form a valve support structure. Alternative valve support structures known in the art for fixing valve leaflets to posts may also be employed.

Posts 32 may be coupled to anchor braid 30 via one or more distal holes 36b. For example, anchor braid 30 may be woven through holes 36b, or a suture or wire may be routed through holes 36b and tied to the braid. Yet another proximal hole (not shown) in post 32 serves as an anchor lock element that interfaces with the anchor lock element provided by buckle 34 to form lock 40. Buckles 34 may likewise be attached to anchor braid 30 via weaving or suturing.

Alternative locks may be used to lock the anchor of the present invention in the foreshortened configuration, as shown, e.g., in Figures 26D-26F. Preferably, a lock of the present invention can have multiple locking options such that locking can confer a plurality of amounts of expansion. Furthermore, the locking option can be employed asymmetrically to confer non-cylindrical shapes to the anchor. In Figure 26D, lock 40' comprises male lock element 44 disposed on post 32 and anchor lock element 34 disposed on braided anchor 30. Anchor lock element 34 illustratively comprises triangular protrusion or eyelet 42 of anchor 30. The triangular shape of female lock element 42 may facilitate mating of male lock element 44 with the female lock element without necessitating deformation of the male lock element. One or more holes 45 may be provided through post 32, e.g., for releasably attaching an actuator 106b to the post.

In Figure 26E, lock 40" comprises alternative male lock element 44' having multiple in-line arrowheads 46 along posts 32. Each arrowhead comprises resiliently deformable appendages 48 to facilitate passage through female lock element 42', which illustratively comprises a rounded eyelet. Appendages 48 optionally comprise holes 49, such that releasable lock prevention mechanism 47, illustratively a control wire, may pass through the holes to constrain the appendages in the deformed configuration. To actuate lock 40", one or more arrowheads 46 of male lock element 44' are drawn through female lock element 42', e.g., via a post/lock actuator, and the lock prevention mechanism is removed from holes 49, thereby causing appendages 48 to resiliently expand and actuate lock 40".

Advantageously, providing multiple arrowheads 46 along posts 32 yields a ratchet that facilitates in-vivo determination of a degree of foreshortening and expansion imposed upon anchor 30. Furthermore, optionally constraining appendages 48 of arrowheads 46 via mechanism 47 prevents actuation of lock 40" (and thereby deployment of apparatus 10) even after male element 44' has been advanced through female element 42'. Only after a medical practitioner has removed lock prevention mechanism 47, which constrains appendages 48, is lock 40" fully engaged and is deployment no longer reversible.

Lock 40'" of Figure 26F is similar to lock 40" of Figure 26E, except that holes 49 on appendages 48 have been eliminated, and the lock prevention mechanism comprises overtube or cover 47. Overtube 47 constrains appendages 48 to prevent locking until a medical practitioner has determined that apparatus of the present invention has been foreshortened and positioned adequately at a treatment site. Lock 40'" may, for example, be actuated by applying a proximally-directed force to actuator 106b. Actuator 106b illustratively comprises a control wire releasably disposed through hole 45 in post 32. Lock prevention mechanism 47 then is withdrawn proximally relative to anchor 30, which causes the appendages to resiliently expand, thereby fully actuating lock 40"'.

With reference now to Figures 27A-F, a method of endovascularly replacing a patient's diseased aortic valve is provided. The method involves endovascularly delivering an anchor/valve apparatus and properly positioning such apparatus via positive registration with the patient's native valve leaflets. Registration with the native valve leaflet preferably occurs using the leaflet engagement elements.

In Figure 27A, modified delivery system 100' delivers apparatus 10 to diseased aortic valve AV within sheath 110. Apparatus 10 is delivered in a collapsed delivery configuration.

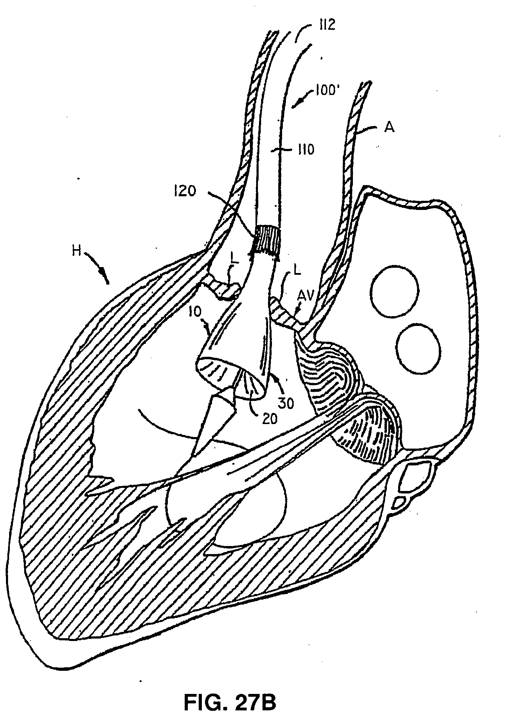

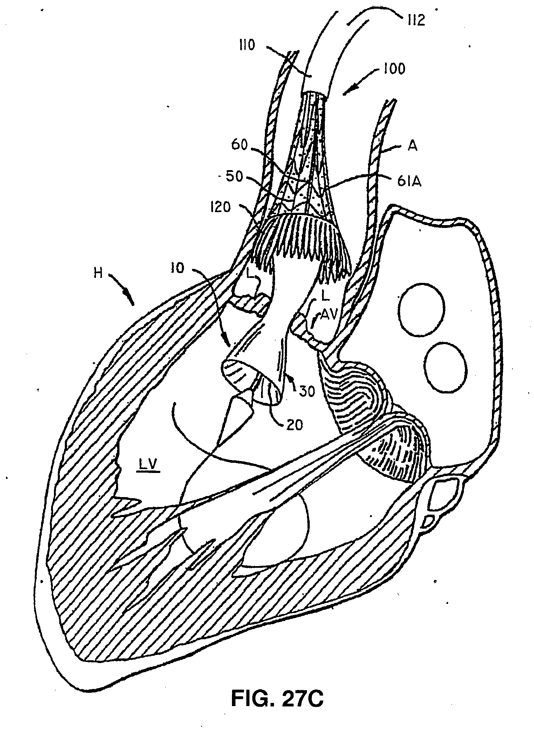

As seen in Figures 27B and 27C, apparatus 10 is deployed from lumen 112 of sheath 110, for example, under fluoroscopic guidance. Sheath 110 includes at its distal end leaflet engagement elements 120. Upon deployment, anchor 30 of apparatus 10 dynamically self-expands to a partially deployed configuration. This causes elements 60 to also dynamically expand, as well as membrane filter (or braid) 61A and leaflet engagement elements 120. As when deployed via delivery system 100, deployment of apparatus 10 via delivery system 100' is fully reversible until locks 40 have been actuated.

Thus, delivery system 100' comprises leaflet engagement element 120, which preferably self-expands along with anchor 30. In preferred embodiments, the distal end of leaflet engagement elements 120 expands a greater radial distance than anchor 30. Moreover, engagement elements 120 maybe disposed between elements 60 of delivery system 100' and lip region 32 of anchor 30. However, leaflet engagement elements 120 may also be disposed on the proximal end of an anchor (as is illustrated in Figure 28). Leaflet engagement elements 120 releasably engage the anchor. As seen in Figure 27C, the leaflet engagement elements 120 are initially deployed proximal of the patient's native valve leaflets L. Apparatus 10 and element 120 then may be advanced/dynamically repositioned until engagement element positively registers against the leaflets, thereby ensuring proper positioning of apparatus 10. The leaflet engagement element engages with the proximal edges of the native valve leaflets and/or the commissural attachments. The leaflet engagement element need not extend all the way to the distal edge of the native leaflets (the leaflet pockets). In preferred embodiments, a leaflet engagement element length is less than about 20 mm, more preferably less than about 15 mm, or more preferably less than about 10 mm. Once leaflet engagement element 120 is registered against the native valve leaflets and/or commissural attachments, apparatus 10 deploys substantially distal to the coronary ostia of the heart.

In any of the embodiments herein, delivery system 100' can include filter structure 61A (e.g., filter membrane or braid) as part of push elements 60 to act as an embolic protection element. Emboli can be generated during manipulation and placement of anchor from either diseased native leaflet or surrounding aortic tissue and can cause blockage. Arrows 61B in Figure 27C show blood flow through filter structure 61A where blood is allowed to flow but emboli is trapped in the delivery system and removed with it at the end of the procedure.

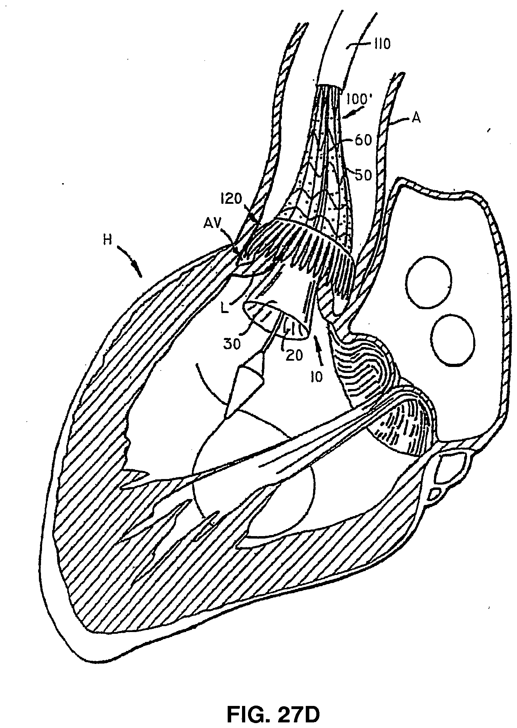

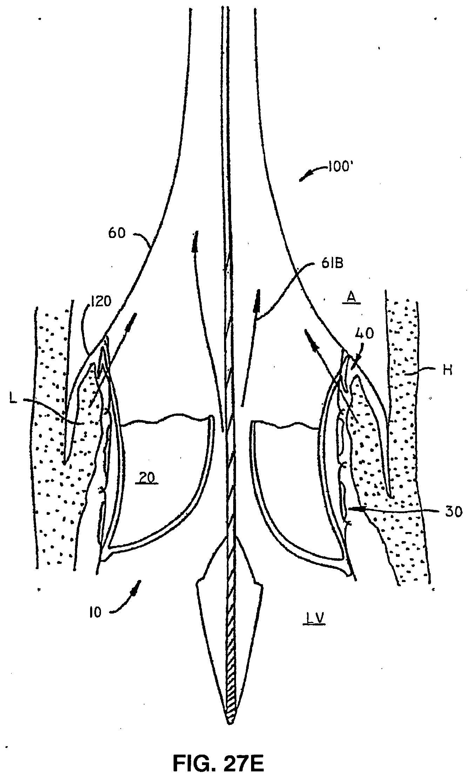

Active foreshortening may be imposed upon anchor 30 while element 120 is disposed proximal of the leaflets, as is illustrated in Figure 27D. Active foreshortening can be accomplished by actuating distal anchor actuation elements (e.g., elements 50) and/or proximal anchor actuation elements (e.g., elements 60). Upon positive registration of element 120 against leaflets L, element 120 precludes further distal migration of apparatus 10 during additional foreshortening, thereby reducing a risk of improperly positioning the apparatus. Figure 27E details engagement of element 120 against the native leaflets.

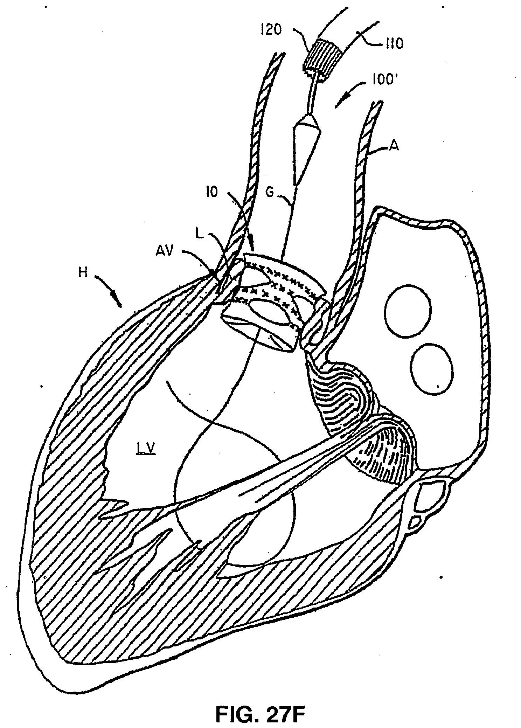

As seen in Figure 27F, once apparatus 10 is fully deployed, anchor 30 may be locked (reversibly or irreversibly). Subsequently, structure 61A leaflet engagement, elements 120, elements 50 and/or elements 60 may be decoupled from the apparatus, and delivery system 100' may be removed from the patient, thereby completing the procedure.

Figure 28 illustrates an alternative embodiment of the apparatus of Figures 27A-F described above, wherein leaflet engagement elements 120 are coupled to anchor 30 of apparatus 10' rather than to delivery system 100. In the embodiment illustrated in Figure 28, leaflet engagement elements 120 remain implanted near the patient's native heart valve after the deployment of apparatus 10' and removal of delivery system 100. Leaflets L may be sandwiched between the proximal region of anchor 30 and leaflet engagement element 120 in the fully deployed configuration. In this manner, element 120 positively registers apparatus 10' relative to the leaflets L and precludes distal migration of the apparatus over time.

Figures 29A-29C illustrate another embodiment for endovascularly delivering an apparatus of the present invention. In Figure 29A, a catheter 600 is delivered percutaneously in a retrograde fashion to the aortic valve. The catheter passes through the native aortic valve before an operator actuates the unseathing of the anchor/valve apparatus. As the sheathing catheter is pulled proximally out of the native valve, anchor 30 and replacement valve 20 become unsheathed. Immediately the portion of the unsheathed anchor 30 dynamically self-expands to its "at rest" position, and replacement valve 20 within the anchor regains an uncollapsed structure, allowing it to begin to function. In preferred embodiments in its "at rest" position, anchor 30 presses against the native leaflets limiting blood from flowing in between the anchor and leaflet. Also, in preferred embodiments, anchor 30 portions relatively adjacent to the valve is externally covered by a seal 60, more preferably the entire exterior contour of anchor 30 excluding the leaflet engagement elements is externally covered by a seal, or more preferably the entire contour of anchor 30 including the external face of the leaflet engagement elements is externally covered by a seal. A seal can be composed of any material that prevents or limits the flow of blood through the anchor. In preferred embodiments, a seal is composed of a thin, elastic polymer or any other type of fabric. The seal can be attached by any means known in the art to the anchor and, in some embodiments, to the distal end of the valve. In preferred embodiments, a seal is attached to the anchor by suturing.

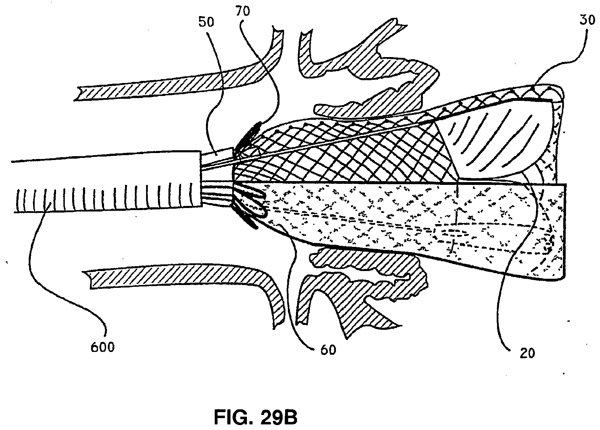

In Figure 29B, as the catheter is further pulled proximally, the proximal end of anchor 30 and fingers 50 are unsheathed. In this embodiment, it is possible to visualize that the seal covers the entire contour of the anchor including the external face of the leaflet engagement element 70. As soon as the proximal end of the anchor is exposed, it also dynamically expands. Furthermore, when fingers 50 become exposed, replacement valve 20 begins to function permitting blood to flow through replacement valve 20, between fingers 50, and around the catheter 600. This also permits blood to flow into the coronary ostias. In other embodiments where the seal does not cover the proximal end of the anchor, the replacement valve can begin to function as soon as the unsealed portion of the anchor is unsheathed. This causes the leaflet engagement elements 70 to radially expand to their heat set position and engage with the native heart leaflets.

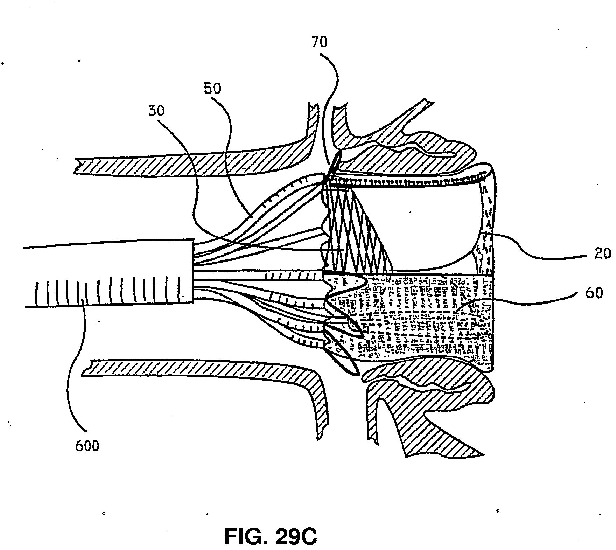

Next, Figure 29C, as the apparatus is actively foreshortened using proximal (e.g., fingers) and/or distal actuators (e.g., elements 55), the leaflet engagement elements positively register with the native valve leaflets. Foreshortening can cause seal 60 to bunch up and create pleats. These pleats can then fill pockets thereby improving the paravalvular seal. In preferred embodiments, wherein the leaflet engagement elements are covered with a seal, at least a portion of the seal is also positioned between the native valve leaflets and the aortic wall. Once the anchor is fully compressed within the aortic valve, the anchor is locked, the fingers and post mandrels are disengaged, and the seal is adapted to further limit blood flow around the replacement valve. The catheter is subsequently'withdrawn, leaving behind valve 20, seal 60 and anchor 70. When fully deployed, the anchor is substantially distal to the coronary ostia of the patient such that it will not interfere with blood flow through the ostia.

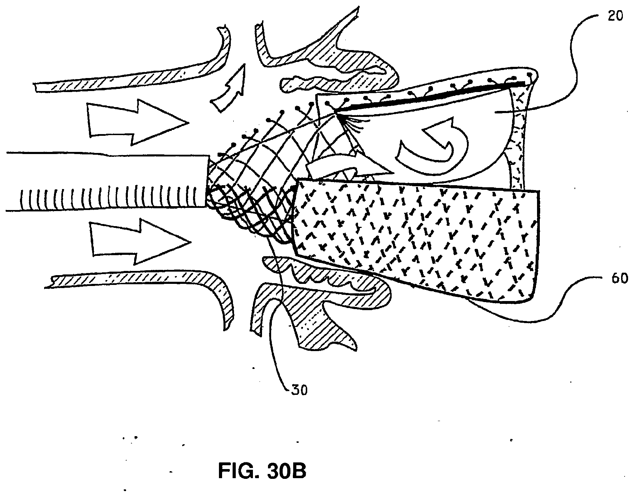

Figures 30A-30B illustrate an embodiment wherein only a distal portion anchor 30 is covered by seal 60 and wherein anchor 30 is only partially deployed since the blood can escape through the proximal end of the anchor braid. As anchor 30 in this embodiment is unsheathed, it presses against the native valve leaflets. At this point replacement valve 20 is functional even though anchor 30 is not fully deployed since blood can escape through the proximal end of the anchor braid. This allows blood to flow through replacement valve 20 and out of holes in the distal end of anchor 30 during systole (Figure 30A) while preventing backflow during diastole (Figure 30B).

Figures 31A-31B illustrate a similar embodiment wherein seal 60 around anchor 30 surrounds the entire contour of anchor 30. In this embodiment, valve 20 does not become functional until both anchor 30 and a portion of fingers 50 are unsheathed. As soon as a portion of fingers 50 is unsheathed, replacement valve 20 is fully functional. This allows blood to flow through replacement valve 20 and anchor 30, out of fingers 50, and around catheter 60 into the aorta and coronary ostias during systole. Similarly, during diastole, replacement valve 20 closes preventing blood backflow from entering the chamber.

In any of the embodiments herein the anchor is preferably a self-expanding anchor braid. Anchor braid of the present invention can be made from one or more wires, more preferably 2-20 wires, more preferably 3-15 wires, or more preferably 4-10 wires. Moreover, the density of the braid can be modified by various forms of weave used.

Figures 22-24 illustrate the process of forming a pleated seal around a replacement valve to prevent leakage. Figure 22 illustrates a fabric seal 380 prior to deployment and foreshortening of the anchor/valve apparatus. In Figure 22, the fabric seal 380 extends from the distal end of valve 20 proximally over anchor 30 during delivery. During deployment, as illustrated in Figure 23, anchor 30 foreshortens and the fabric seal 380 bunches up to create fabric flaps and pockets that extend into spaces formed by the native valve leaflets 382. The bunched up fabric or pleats occur, in particular, when the pockets are filled with blood in response to backflow blood pressure. The pleating can create a seal around the replacement valve. Figure 24 illustrates anchor 30, surrounded by fabric seal 380 in between native valve leaflets 382. In preferred embodiments, at least a portion of a seal is captured between the leaflets and the wall of the heart when the anchor is fully deployed.

## Claims

1. Apparatus for endovascularly replacing a patient's heart valve, the apparatus comprising:
an expandable anchor (30) supporting a replacement valve (20), the anchor having a delivery configuration and a deployed configuration,
**characterized by**
a fabric seal extending from the distal end of the valve (20) proximally over the anchor in the delivery configuration, wherein the seal is bunched up in the deployed configuration,
wherein the apparatus is configured such that during deployment, the expandable anchor (30) foreshortens and the fabric seal bunches up to create fabric flaps and pockets that extend into spaces formed by the native valve leaflets (382).

2. The apparatus of any of the preceding claims, wherein the seal bunches up and creates pleats.

3. The apparatus of any of the preceding claims, wherein the seal comprises a pleated seal.

4. The apparatus of claim 2 or 3, wherein the pleating creates a seal around the replacement valve.

5. The apparatus of any of the preceding claims, wherein the seal bunches up in response to backflow blood pressure.

6. The apparatus of any of the preceding claims, wherein the bunched up fabric or pleats occur in particular when the pockets are filled with blood in response to backflow blood pressure.

7. The apparatus of any of the preceding claims, wherein the expandable anchor has a delivery length in a delivery configuration that is substantially greater than a deployed length in a deployed configuration.

8. The apparatus of any of the preceding claims, wherein the delivery configuration is a collapsed configuration and the deployed configuration is an expanded configuration.

9. The apparatus any of the preceding claims, wherein the anchor self-expands from the delivery configuration.

10. The apparatus any of the preceding claims, wherein the anchor is balloon expandable.

11. The apparatus of any of the preceding claims, wherein at least a portion of the seal is adapted to be captured between native valve leaflets and a wall of the patient's heart when the anchor and replacement valve are fully deployed.

## Patentansprüche

1. Vorrichtung zum endovaskulären Ersetzen der Herzklappe eines Patienten, wobei die Vorrichtung umfasst:
eine expandierbare Befestigung (30), die eine Ersatzklappe (20) stützt, wobei die Befestigung eine Zuführkonfiguration und eine eingesetzte Konfiguration hat,
**gekennzeichnet durch**
eine Abdichtung (380) aus Gewebe, die sich vom distalen Ende der Klappe (20) in proximaler Richtung hin über die Befestigung in der Zuführkonfiguration erstreckt, wobei die Abdichtung in der eingesetzten Konfiguration aufgebauscht ist,
wobei die Vorrichtung derart ausgelegt ist, dass sich während des Einsetzens die expandierbare Befestigung (30) verkürzt und die Abdichtung aus Gewebe aufbauscht, um Laschen und Taschen aus Gewebe zu bilden, die sich in Räume erstrecken, die von den nativen Klappensegel (382) gebildet werden.

2. Die Vorrichtung nach einem der vorhergehenden Ansprüche, wobei die Abdichtung aufbauscht und Falten bildet.

3. Die Vorrichtung nach einem der vorhergehenden Ansprüche, wobei die Abdichtung eine Abdichtung mit Falten umfasst.

4. Die Vorrichtung nach Anspruch 2 oder 3, wobei die Falten eine Abdichtung um die Ersatzklappe herum bilden.

5. Die Vorrichtung nach einem der vorhergehenden Ansprüche, wobei die Abdichtung in Reaktion auf rückfließenden Blutdruck aufbauscht.

6. Die Vorrichtung nach einem der vorhergehenden Ansprüche, wobei das aufgebauschte Gewebe oder die Falten insbesondere auftreten, wenn die Taschen in Reaktion auf rückfließenden Blutdruck mit Blut gefüllt werden.

7. Die Vorrichtung nach einem der vorhergehenden Ansprüche, wobei die expandierbare Befestigung eine Zuführlänge in einer Zuführkonfiguration hat, die wesentlich größer ist als die eingesetzte Länge in einer eingesetzten Konfiguration.

8. Die Vorrichtung nach einem der vorhergehenden Ansprüche, wobei die Zuführkonfiguration eine kollabierte Konfiguration und die eingesetzte Konfiguration eine expandierte Konfiguration ist.

9. Die Vorrichtung nach einem der vorhergehenden Ansprüche, wobei die Befestigung aus der Zuführkonfiguration selbstexpandiert.

10. Die Vorrichtung nach einem der vorhergehenden Ansprüche, wobei die Befestigung ballonexpandierbar ist.

11. Die Vorrichtung nach einem der vorhergehenden Ansprüche, wobei zumindest ein Teil der Abdichtung ausgelegt ist, um zwischen den nativen Klappensegeln und der Wand eines Herzens eines Patienten festgehalten zu werden, wenn die Befestigung und die Ersatzklappe vollständig eingesetzt sind.

## Revendications

1. Appareil de remplacement par voie endovasculaire d'une valvule cardiaque d'un patient, l'appareil comportant :
un ancrage (30) extensible qui supporte une valvule de remplacement (20), l'ancrage ayant une configuration d'acheminement et une configuration déployée,
**caractérisé par**
un joint d'étanchéité (380) en tissu qui s'étend de l'extrémité distale de la valvule (20) dans une direction proximale sur l'ancrage dans la configuration d'acheminement, dans lequel le joint d'étanchéité est tassé dans la configuration déployée,
l'appareil étant adapté de telle sorte que, pendant le déploiement l'ancrage (30) extensible se raccourcit et que le joint d'étanchéité en tissu se tasse pour créer des rabats et des poches en tissu s'étendant dans des espaces formés par les feuillets de la valvule naturelle (382).

2. Appareil selon l'une quelconque des revendications précédentes, dans lequel le joint d'étanchéité se tasse et crée des plis.

3. Appareil selon l'une quelconque des revendications précédentes, dans lequel le joint d'étanchéité comporte un joint d'étanchéité plissé.

4. Appareil selon la revendication 2 ou 3, dans lequel le plissement crée un joint d'étanchéité autour de la valvule de remplacement.

5. Appareil selon l'une quelconque des revendications précédentes, dans lequel le joint d'étanchéité se tasse en réponse à une pression sanguine de reflux.

6. Appareil selon l'une quelconque des revendications précédentes, dans lequel le tissu ou les plis tassés surviennent en particulier lorsque les poches sont remplies de sang en réponse à une pression sanguine de reflux.

7. Appareil selon l'une quelconque des revendications précédentes, dans lequel l'ancrage extensible a une longueur d'acheminement dans une configuration d'acheminement qui est sensiblement supérieure à une longueur déployée dans une configuration déployée.

8. Appareil selon l'une quelconque des revendications précédentes, dans lequel la configuration d'acheminement est une configuration repliée et la configuration déployée est une configuration en extension.

9. Appareil selon l'une quelconque des revendications précédentes, dans lequel l'ancrage est auto-extensible depuis la configuration de déploiement.

10. Appareil selon l'une quelconque des revendications précédentes, dans lequel l'ancrage est extensible par ballonnet.

11. Appareil selon l'une quelconque des revendications précédentes, dans lequel au moins une partie du joint d'étanchéité est conçue pour être prise entre les feuillets de la valvule naturelle et une paroi du cœur du patient lorsque l'ancrage et la valvule de remplacement sont entièrement déployés.
